Europäisches Patentamt

European Patent Office

Office européen des brevets

(11) Publication number: **0 266 015 B1**

# EUROPEAN PATENT SPECIFICATION

(45) Date of publication of patent specification: **04.12.91**  (51) Int. Cl.⁵: **B01J  23/68**, C07D 301/10

(21) Application number: **87202103.5**

(22) Date of filing: **30.10.87**

(54) **Ethylene oxide catalyst and process for the catalytic production of ethylene oxide.**

(30) Priority: **31.10.86 US 926025**
      **31.10.86 US 926026**

(43) Date of publication of application:
      **04.05.88 Bulletin  88/18**

(45) Publication of the grant of the patent:
      **04.12.91 Bulletin  91/49**

(84) Designated Contracting States:
      **AT BE CH DE ES FR GB GR IT LI LU NL SE**

(56) References cited:
      **EP-A- 0 172 565**
      **US-A- 3 449 078**
      **US-A- 4 350 616**
      **US-A- 4 548 921**

(73) Proprietor: **SHELL INTERNATIONALE RE-
      SEARCH MAATSCHAPPIJ B.V.**
      **Carel van Bylandtlaan 30**
      **NL-2596 HR Den Haag(NL)**

(72) Inventor: **Lauritzen, Ann Marie**
      **16810 Gaelic Lane**
      **Houston Texas 77084(US)**

(74) Representative: **Tuijn, Jan Warnaar et al
      Shell Internationale Research Maatschappij
      B.V., Patents, Licensing & Trade Marks Di-
      vision, P.O. Box 302
      NL-2501 CH The Hague(NL)**

## Description

This invention relates to catalyst compositions, suitable for the catalytic manufacture of ethylene oxide from ethylene and oxygen, comprising a support, silver and rhenium.

Supported silver catalysts have long been used in the vapour phase conversion of ethylene and oxygen to ethylene oxide. The use of small amounts of the alkali metals, K, Rb and Cs, were noted as useful promoters in supported silver catalysts in U.S. patents numbered US-A 3,962,136 and US-A 4,010,115.

US-A 3,844,981; 3,962,285; and GB-A 1,325,715 disclose the use of silver-rhenium ethylene oxide catalysts. In these patents a high surface area silver derivative such as silver oxide is impregnated with a rhenium solution and subsequently reduced to provide metallic rhenium alloyed with the silver, but no support is being employed. US-A 4,548,921 discloses the use of rhenium in silver-supported ethylene oxide catalysts, the rhenium is first placed on the support in the form of finely divided metal particles and the silver is subsequently deposited on the outer surface of the particles. US-A 3,972,829 discloses a method for distributing catalytically active metallic components on supports using an impregnating solution of catalyst precursor compound and an organic thioacid or a mercaptocarboxylic acid. Catalytically active metals include metals of Groups IVA, 1B, VIB, VIIB and VIII, including rhenium and which may be in either the oxidized or reduced state. However, promoting amounts of rhenium in combination with silver and promoter amounts of alkali metal on a porous refractory support are not suggested. US-A 4,459,372, discloses the use of rhenium metal in combination with a surface metallated (using Ti, Zr, Hf, V, Sb, Pb, Ta, Nb, Ge and/or Si) alumina or silica. None of these references disclose the use of a promoting amount of rhenium which is present on a silver-based, alkali-doped supported catalyst.

US-A 3,449,078 discloses presulphided hydrocarbon conversion catalysts for production of hydrogen, which comprise silver, a support, rhenium and potassium, use of an alumina support having a surface area of 255 $m^2/g$ has been exemplified. US-A 4,350,616 is concerned with ethylene oxide catalysts comprising silver, a support, cesium, but no rhenium. The same applies to EP-A 172,565.

Whilst alkali-doped supported silver catalyst compositions are presently commercially available and have a good performance particularly as far as selectivity is concerned, the elaborate patent literature, which discloses a constant stream of new inventions, shows that the search for improved catalyst compositions has not come to an end as yet. In particular, conceiving catalysts that have an optimum selectivity performance above the level of alkali-doped catalysts, preferably in combination with an improved stability (longevity) remains a problem that presently has not been solved in an entirely satisfactory manner.

The present invention provides catalyst compositions, suitable for the catalytic manufacture of ethylene oxide from ethylene and oxygen, containing silver and a support. They are characterized in that they comprise a support having a surface area of less than 20 $m^2/g$ and a promoting amount of rhenium or a compound thereof and a promoting amount of at least one further metal or a compound thereof.

In the most preferred embodiment the further metal is potassium, rubidium, cesium or mixtures thereof and is present in an amount ranging from 20 to 1500 ppm by weight of the total catalyst and the rhenium is present in an amount ranging from 0.2 to 5, more preferably from 0.3 to 3.5 millimoles of rhenium per kilogram of total catalyst. The rhenium may conveniently be a form of rhenium which is extractable in a dilute aqueous alkali metal hydroxide solution, particularly a 20 millimolar sodium hydroxide solution. In a further preferred embodiment the instant combination of silver, alkali metal promoter, rhenium promoter and support affords higher selectivities, particularly higher initial selectivities to ethylene oxide at a given oxygen conversion level than is obtained under the same reaction conditions with the same combination of silver, support and none or one of the promoters selected from rhenium and further metal.

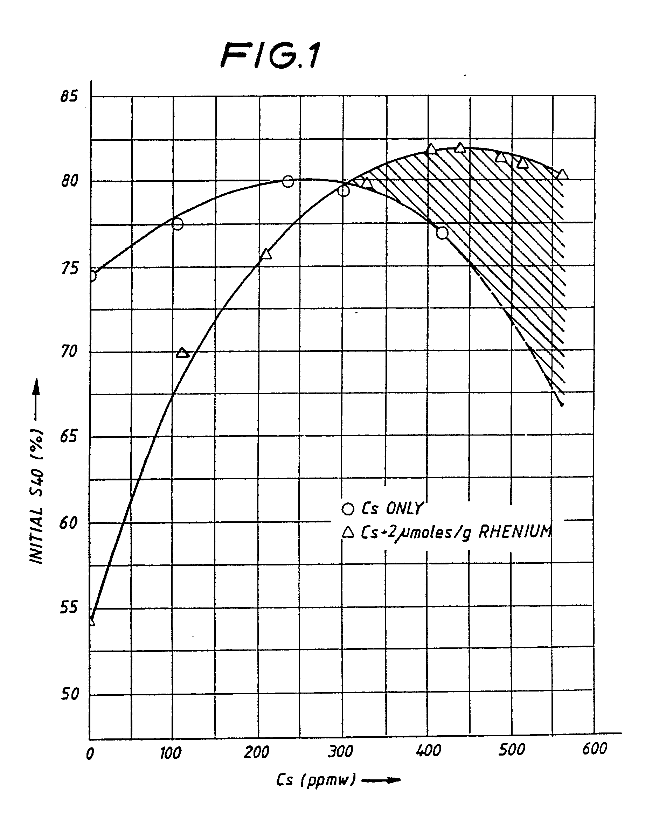

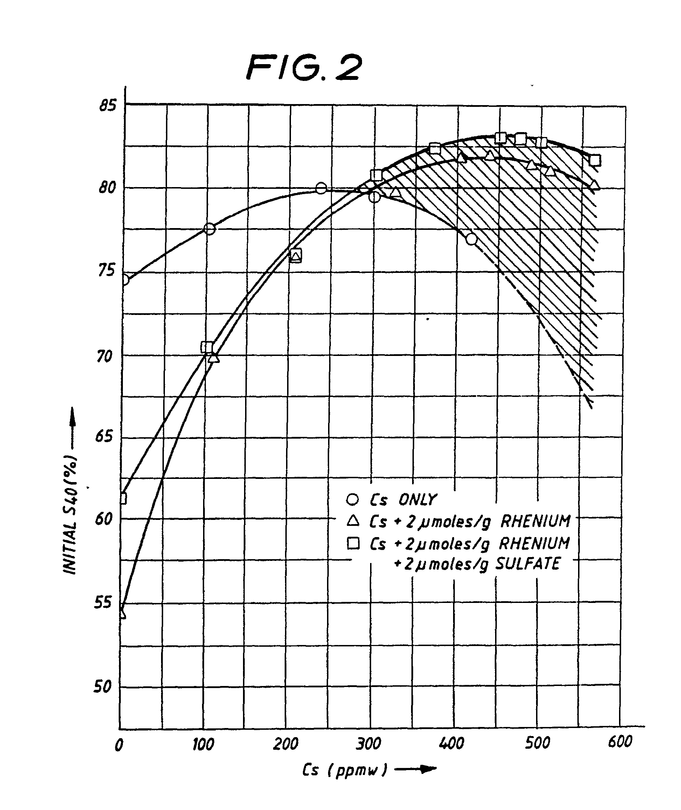

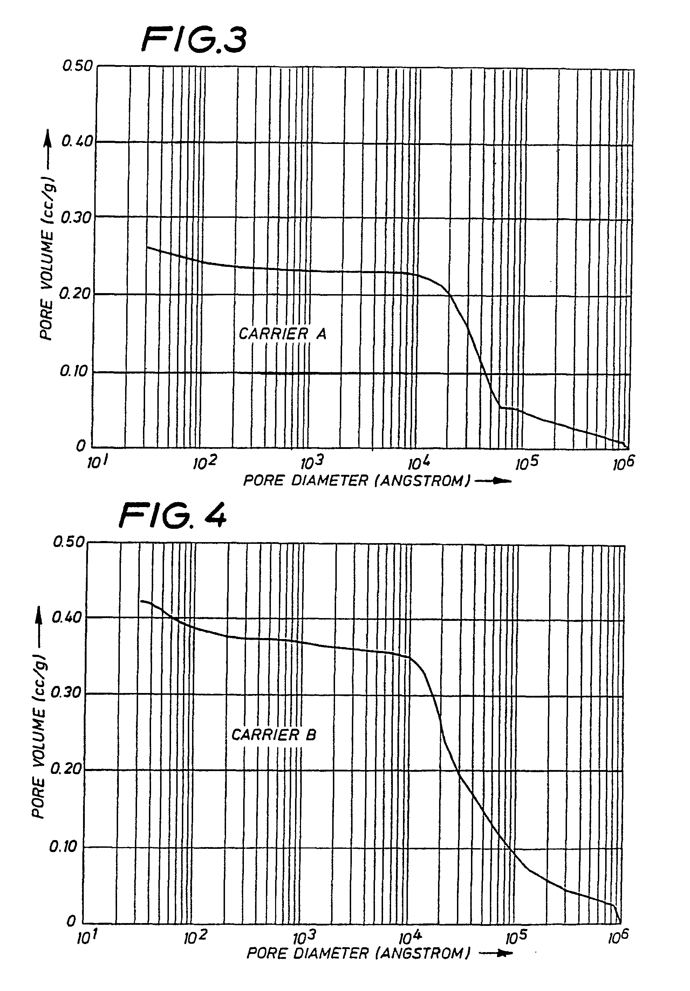

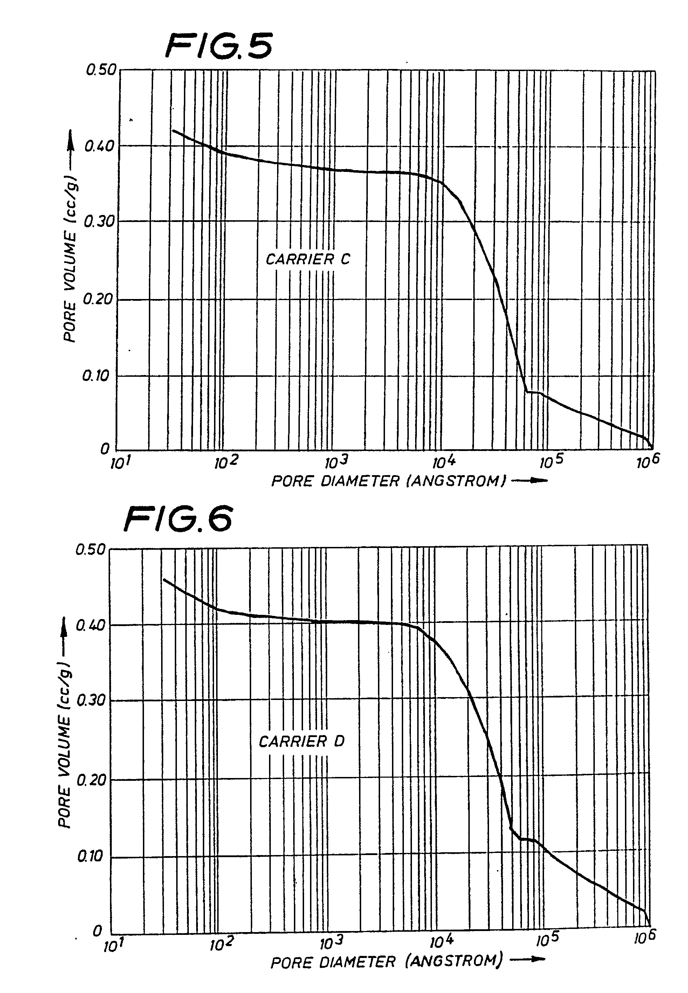

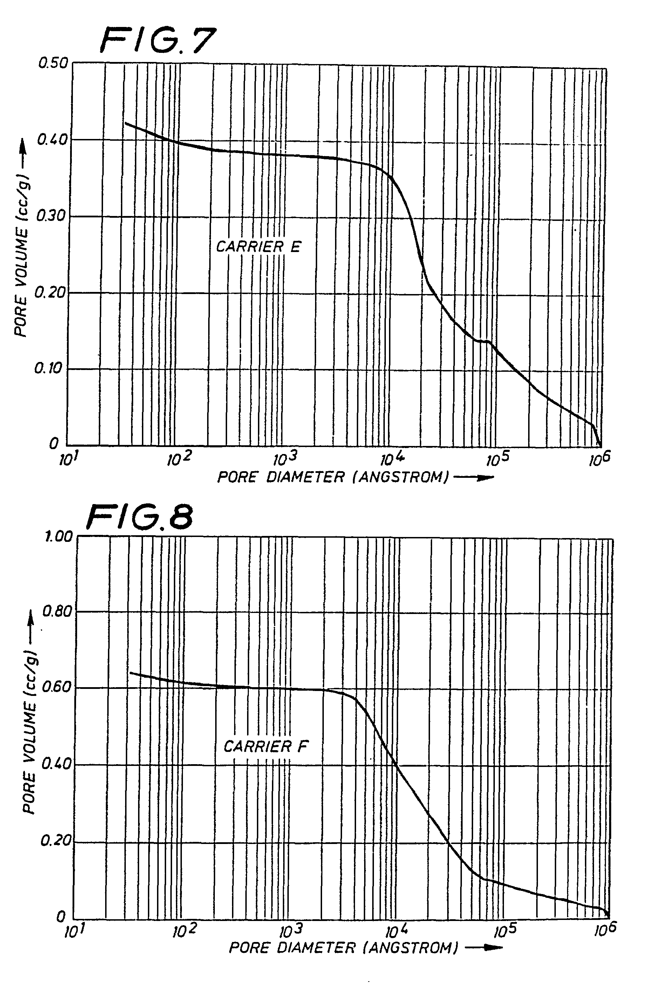

Figure 1 shows optimized initial selectivity versus cesium promoter concentration for a catalyst of the instant invention containing rhenium and for a catalyst not containing rhenium thereby illustrating the enhanced initial selectivity obtained with the instant catalyst. Figure 2 shows further increased selectivity achievable with catalysts that include sulphur or a sulphur compound as an additional further promoter (copromoter). Figures 3-8 show pore size distribution curves for carriers A-F of Table 1.

Generally, in the vapour phase reaction of ethylene with oxygen to produce ethylene oxide, the ethylene is present in at least a double amount (on a mol basis) compared with oxygen, but frequently is often much higher. Therefore the conversion is calculated according to the mol percentage of oxygen which has been used in the reaction. The oxygen conversion is dependent on the reaction temperature which latter is a measure of the activity of the catalyst employed. The value $T_{40}$ indicates the temperature at 40 mol percent conversion of the oxygen in the reactor and the value T is expressed in °C. This temperature is higher when the conversion of oxygen is higher. Moreover this temperature is strongly dependent on the employed catalyst and the reaction conditions. The selectivity (to ethylene oxide) indicates the molar amount of ethylene oxide in the reaction product compared with the total molar amount of ethylene converted. Herein the selectivity is indicated as $S_{40}$, which means the selectivity at 40 mol percent oxygen conversion. The selectivity of silver-based ethylene oxide catalysts can decrease over a period of time of usage. When comparing the selectivity performance of various silver-based ethylene oxide catalysts, it is important that the selectivity value be measured at approximately the same period of time of usage under the same or similar reaction conditions. As used herein, "initial selectivity" will refer to the selectivity of ethylene oxide catalysts when measured at a given constant oxygen conversion level of 40% at a gas hourly space velocity of approximately 3300 and when measured after the catalyst has been placed on stream for about 16 ± 4 hours. Unless otherwise noted, all selectivities that are provided in the examples provided herein are initial selectivities.

In broad general terms the catalysts of the instant invention may suitably be prepared by impregnating supports, particularly porous refractory supports, with silver ions, or compound(s), complex(es) and/or salt(s) dissolved in a suitable solvent sufficient to cause deposition on the support of, in particular, from 1 to 30 percent by weight, preferably 5 to 20 precent by weight, basis total catalyst, of silver; the thus impregnated carrier is then separated from the solution and the deposited silver compound is reduced to metallic silver. Also deposited on the support either prior to, coincidentally with, or subsequent to the deposition of the silver will be suitable ions, or compound(s) and/or salt(s) of further metal or metal compound dissolved in a suitable solvent. Also deposited on the carrier either prior to, coincidentally with, or subsequent to the deposition of the silver and/or further metal will be suitable rhenium ions, or compound(s), complex(es) and/or salt(s) dissolved in an appropriate solvent. Detailed preparative techniques are discussed herein. The weight ratio of silver to rhenium in the catalyst is preferably more than 1.0.

The support or carrier employed in these catalysts in its broadest aspects is selected from the larger number of conventional, porous refractory catalyst carriers or support materials which are considered relatively inert in the presence of the ethylene oxidation feeds, products and reaction conditions. Such conventional materials are known to persons skilled in the art and may be of natural or synthetic origin and preferably are of a macroporous structure, that is, a structure having a B.E.T. surface area below 20 m²/g. Very suitable supports comprise those of aluminous composition. Examples of supports that have been used as supports for different catalysts and which could, it is believed, be used as supports for ethylene oxide catalysts are the aluminium oxides (including the materials sold under the trade name "Alundum"), charcoal, pumice, magnesia, zirconia, kieselguhr, fullers' earth, silicon carbide, porous agglomerates comprising silica and/or silicon carbide, silica, magnesia, selected clays, artificial and natural zeolites and ceramics. Refractory supports particularly useful in the preparation of catalysts in accordance with this invention comprise the aluminous materials, in particular those comprising alpha alumina. In the case of alpha alumina-containing supports, preference is given to those having a specific surface area as measured by the B.E.T. method of from 0.01 m²/g to 10 m²/g, preferably 0.02 to 15, more preferably from 0.05 to 5, most preferably from 0.1 to 3 m²/g, and a water pore volume as measured by conventional water absorption techniques of from 0.1 to 0.75 ml/g. The B.E.T. method for determining specific surface area is described in detail in Brunauer, S., Emmet, P.Y. and Teller, E., J. Am. Chem. Soc., 60, 309-16 (1938).

Certain types of alpha alumina-containing supports are particularly preferred. These alpha alumina supports have relatively uniform pore diameters and are more fully characterized by having (1) B.E.T. specific surface areas of from 0.1 m²/g to 3.0 m²/g, preferably 0.1 m²/g to 2.0 m²/g and (2) water pore volumes of from 0.10 ml/g to 0.75 ml/g, preferably from 0.25 ml/g to 0.55 ml/g. Typical properties of some supports found useful in the instant invention are shown in Table 1. Suitable manufacturers of carriers comparable to those in Table 1 include Norton Company and United Catalysts, Inc.

TABLE 1

| Carrier | A | B | C | D | E | F |
|---|---|---|---|---|---|---|
| B.E.T. Surface Area, $m^2/g$ [a] | 0.21 | 0.42 | 0.42 | 0.48 | 0.57 | 2.06 |
| Water Pore Volume, ml/g | 0.26 | 0.36 | 0.41 | 0.49 | 0.44 | 0.65 |
| Crush Strength, FPCS, lbs [b] | 100% *25 lbs 5* | 97% *15 6.8* | Avg 21 Range 15-30 *6.8 -13 6* | 90% *14 6.7* | 90% *15 6.8* | No Data |
| Total Pore Volume, Hg, ml/g [c] | 0.26 | 0.42 | 0.42 | 0.46 | 0.42 | 0.65 |
| Average Pore Diameter, A Hg [c] | 620 | 560 | 640 | 550 | 770 | 1000 |
| Median Pore Diameter, Hg, microns [c,e] | 3.7 | 2.7 | 3.4 | 3.4 | 2.4 | 2.5 |
| Percent Pore Volume in Pores Greater Than 350 A [c] | 90.0% | 88.5% | 89.5% | 89.1% | 91.5% | 94.1% |
| Percent Pore Volume in Pores Greater Than 1 Micron [c] | 87.0% | 82.5% | 83.4% | 82.3% | 83.5% | 61.0% |
| % Wt. Alpha Alumina | 99.5 | 98 | 98.5 | 98.5 | 98 | 70-75 |
| Water Leachable Na, ppmw | 12 | 53 | 21 | 24 | 18 | No Data |
| Acid-Leachable Na, ppmw | 40 | 96 | 87 | 51 | 45 | No Data |
| Water-Leachable K, ppmw | 5 | 22 | 21 | 22 | 10 | No Data |
| Acid-Leachable Fe, ppmw | 2 | 5 | No Data | 1 | 5 | No Data |
| % Wt. $SiO_2$ | .5 | 2 | 1.5 | 15 | 2 | 25-30 |

(a) Method of Brunauer, Emmet and Teller, loc. cit.

(b) Flat Plate Crush Strength, single pellet.

(c) Determined by mercury intrusion to 55,000 psia using Micrometrics Autopore 9200 or 9210 (130° Contact angle, 0.473 N/m surface tension of Hg).

(e) Median pore diameter represents the pore diameter wherein 50% of the total pore volume is found in pores having less than (or greater than) the median pore diameter.

Pore size distribution curves measured by mercury intrusion techniques noted in footnote (c) above in Table 1 for carriers A-F are shown in Figures 3-8.

Of the carriers listed in Table 1, B and D are preferred because they provide catalysts which show better overall initial performance in terms of initial selectivity and initial activity.

Regardless of the character of the support or carrier used, it is preferably shaped into particles, chunks, pieces, pellets, rings, spheres, wagon wheels and the like of a size suitable for employment in fixed bed reactors. Conventional commercial fixed bed ethylene oxide reactors are typically in the form of a plurality of parallel elongated tubes (in a suitable shell) approximately 1.8 to 6.8 cm O.D. and 1.3 to 6.4 cm I.D. and 4.5-13.5 m long filled with catalyst. In such reactors, it is desirable to employ a support formed into a rounded shape, such as for example, spheres, pellets, rings, tablets and the like, having diameters of from 0.25 cm to 2 cm.

Particular supports may be selected having differing properties such as surface area and pore volume in order to provide particular catalytic properties. With regard to surface area (B.E.T.) possible lower limits are, for example, 0.01, 0.05, and 0.2 $m^2/g$ and possible upper limits are, for example, 1, 3, 5, 10, 15 and 20 $m^2/g$. With regard to water pore volume, possible lower limits are, for example, 0.05, 0.2 and 0.35 ml/g and possible upper limits are, for example, about 0.6, and 0.8 ml/g.

The catalysts of the instant invention are prepared by a technique in which the further metal promoters and the rhenium in the form of soluble salts and/or compounds are deposited on the catalyst and/or support prior to, simultaneously with, or subsequent to the deposition of the silver and each other. The further metals may be deposited at one step of the process and the rhenium at a different step or steps. The preferred method is to deposit silver, further metal and rhenium simultaneously on the support, that is, in a single impregnation step, although it is believed that the individual or concurrent deposition of the further metal and rhenium prior to and/or subsequent to the deposition of the silver produces suitable catalysts.

Although the further metals may exist in a pure metallic state, they are not in that form suitable for use. They are utilized as ions or salts or compounds of metals dissolved in a suitable solvent for impregnation purposes. The porous carrier is impregnated with a solution of metal promoter ions, salt(s) and/or compound(s) before, during or after impregnation or deposition of the silver ions, salt(s), complex(es) and/or compound(s) has taken place. A further metal promoter may even be deposited on the carrier after reduction to metallic silver has taken place. The promoting amount of further metal utilized will depend on several variables, such as, for example, the surface area and pore structure and surface chemical properties of the carrier used, silver content of the catalyst and the particular ions used in conjunction with the further metal cation or rhenium and amounts of rhenium present. The amount of further metal promoter deposited upon the support or present on the catalyst generally lies between 10 and 3000, preferably between 15 and 2000 and more preferably between 20 and 1500 parts by weight (calculated as metal) per million parts by weight of total catalyst. Most preferably, the amounts range between 50 and 1000 parts per million by weight of the total catalyst. The degree of benefit obtained within the above-defined limits will vary depending upon particular properties and characteristics, such as, for example, reaction conditions, catalyst preparative techniques, surface area and pore structure and surface chemical properties of the carrier utilized, silver content of the catalyst and other compounds, cations or anions present in addition to further metal ions such as the ions added with the further metal or rhenium or compounds remaining from the impregnating solution, and the above-defined limits were selected to cover the widest possible variations in properties and characteristics. The effects of these variations are readily determined by experimentation. The further metal promoters are present on the catalysts in the form of cations (ions) or compounds or complexes or surface compounds or surface complexes rather than as the extremely active free metals. Without intending to limit the scope of the invention, it is believed that the further metal promoters are present as oxidic compounds. More particularly, it is believed that the further metal compounds are probably in the form of mixed surface oxides or double surface oxides or complex surface oxides with the aluminium of the support and/or the silver of the catalyst, possibly in combination with species contained in or formed from the reaction mixture such as chlorides or carbonates or residual species from the impregnation solution(s).

From the above it will be clear that the catalysts of this invention are concerned with the different types of promoters, the first being rhenium and the second being the further metal compound. The said order "first" and "second" does not refer to the importance of the contribution towards improving the performance of the ethylene oxide catalyst. This importance may be reversed i.e. the contribution towards improving catalyst selectivity of the second promoter may be significantly larger than that of rhenium.

A large scope of various further metal promoter is envisaged in this invention, suitable second promoters may comprise metals such as molybdenum, tungsten, chromium, titanium, hafnium, thorium, zirconium, vanadium, thallium, tantalum, niobium, gallium and germanium. Compounds of molybdenum, tungsten or chromium have the ability to perform as copromoter to a further metal promoter selected from

5

other metals.

Preferred second promoters are selected from the group of earth alkali metals with magnesium, barium and calcium being of special interest. Even more preferred are alkali metals, particularly those selected from the group consisting of potassium, rubidium, cesium and mixtures thereof, with cesium being the best option. In this preferred embodiment, the alkali metals comprise the higher alkali metals. As used herein the term "higher alkali metal" and cognates thereof refers to the alkali metals selected from the group consisting of potassium, rubidium, cesium and mixtures thereof. As used herein, the term "mixtures of alkali metals" or "mixtures of higher alkali metals" or cognates of these terms refers to the use of two or more of the alkali or higher alkali metals, as appropriate, to provide a promoting effect. Non-limiting examples include cesium plus rubidium, cesium plus potassium, cesium plus sodium, cesium plus lithium, cesium plus rubidium plus sodium, cesium plus potassium plus sodium, cesium plus lithium plus sodium, cesium plus rubidium plus potassium plus sodium, cesium plus rubidium plus potassium plus lithium, cesium plus potassium plus lithium and the like. When the alkali metal comprises mixtures of higher alkali metals, at least two of the following are used, potassium, rubidium or cesium. Thus, for example, in the preferred embodiment wherein the higher alkali metal comprises potassium, rubidium, cesium or mixtures thereof, potassium may be used with cesium, or rubidium may be used with cesium, or potassium may be used with rubidium or all three may be used together. Hence, for example when potassium is used with cesium, the weight percent ratio of potassium to cesium will range from 0/100 to 100/0, including all ranges in between such as 20/80, 50/50, 75/25 etc., and similar relationships will apply to other mixtures. A particularly preferred alkali metal promoter is cesium.

It must be clear that the amounts of alkali metal promoters on the catalysts are not necessarily the total amounts of these metals present in the catalyst. They are amounts that have been added to the catalyst by impregnation with suitable solutions of ions, salts and/or compounds and/or complexes of alkali metals. These amounts do not include amounts of alkali metals that are locked into the support, say by calcining, or are not extractable in a suitable solvent such as water or lower alkanol or amine or mixtures thereof and do not provide a promoting effort. It is also understood that the source of the alkali metal promoter ions, salts and/or compounds used to impregnate the catalyst may be the carrier. That is, the carrier may contain extractable amounts of alkali metal that can be extracted with a suitable solvent such as water or lower alkanol, thus preparing an impregnating solution from which the alkali metal ions, salts and/or compounds are deposited or redeposited on the support.

As used herein, the term "compound" refers to the combination of a particular element with one or more different elements by surface and/or chemical bonding, such as ionic and/or covalent and/or coordinate bonding. The term "ionic" or "ion" refers to an electrically charged chemical moiety; "cationic" or "cation" being positive and "anionic" or "anion" being negative. It is understood that ions do not exist in vacuo, but are found in combination with charge-balancing counter ions. The term "oxidic" refers to a charged or neutral species wherein an element in question is bound to oxygen and possibly one or more different elements by surface and/or chemical bonding, such as ionic and/or covalent and/or coordinate bonding. Thus, an oxidic compound is an oxygen-containing compound which also may be a mixed, double or complex surface oxide. Illustrative oxidic compounds include, by way of nonlimiting example, oxides (containing only oxygen as the second element), hydroxides, nitrates, sulfates, carboxylates, carbonates, bicarbonates, oxyhalides, etc., as well as surface species wherein the element in question is bound directly or indirectly to an oxygen either in the substrate or on the surface.

As used herein, the term "promoting amount" of a certain component of a catalyst refers to an amount of that component that works effectively to provide an improvement in one or more of the catalytic properties of that catalyst when compared to a catalyst not containing said component. Examples of catalytic properties include, inter alia, operability (resistance to runaway), selectivity, activity, conversion, stability and yield. It is understood by one skilled in the art that one or more of the individual catalytic properties may be enhanced by the "promoting amount" while other catalytic properties may or may not be enhanced or may even be diminished. It is further understood that different catalytic properties may be enhanced at different operating conditions. For example, a catalyst having enhanced selectivity at one set of operating conditions may be operated at a different set of conditions wherein the improvement shows up in the activity rather than the selectivity and an operator of an ethylene oxide plant will intentionally change the operating conditions in order to take advantage of certain catalytic properties even at the expense of other catalytic properties in order to maximize profits by taking into account feed stock costs, energy costs, byproduct removal costs and the like. The particular combination of silver, support, alkali metal and rhenium of the instant invention will provide an improvement in one or more catalytic properties over the same combination of silver and support and none or one of the promoters selected from rhenium and alkali metal. More preferably an improvement is provided over the same combination of silver, support and second

EP 0 266 015 B1

promoter but not containing a promoting amount of rhenium.

As will be understood, the catalysts of this invention should contain a "catalytically effective amount of silver", which term refers to an amount of silver that provides a measurable conversion of ethylene and oxygen to ethylene oxide.

A preferred embodiment of this invention is concerned with catalysts, as defined herein-before, that in ethylene oxide production are able to produce a selectivity of either a) at least 20% at 20% oxygen conversion level or b) at least 10% at 40% oxygen conversion level. A suitable method for testing the performance of the catalysts is indicated hereinafter in Example 1 and a positive performance, shown in this very test method, can be used as a yardstick for defining catalysts belonging to said preferred embodiment of this invention.

Another preferred embodiment of this invention is concerned with narrowing down the term "effective promoting amount", as used in this specification in relation to amounts of rhenium and/or other promoting metal, to define any such amounts that will lead to an improvement of the performance level of the catalysts irrespective whether this is selectivity, conversion or stability. Here again a suitable method for testing the catalyst is the method disclosed in Example 1 and an improvement shown upon testing catalysts under the conditions of that very method can be used as a yardstick for defining catalyst belonging to the last-mentioned preferred group of catalysts. It will be clear that the envisaged improvement is an improvement obtained in comparison with the performance shown at the same reaction conditons of the same combination of silver and support and none or one promoter selected from rhenium and further promoting metal.

More preferred improvements are those obtained with catalysts comprising rhenium in comparison with the performance shown at the same reaction conditions, of the same combination of silver, support and further metal promoting compound, but not containing rhenium.

The most preferred catalysts within this group are those that can achieve an improvement of the selectivity, preferably by at least 0.1 points, particularly at least 0.3 point s. Another most preferred group of catalysts comprises those that can achieve an improvement in both selectivity and stability or in the balance of selectivity and stability.

The carrier is also impregnated with rhenium ions, salt(s), compound(s) and/or complex(es). This may be done at the same time that the alkali metal promoter is added, before or later; or at the same time that the silver is added or before or later. Preferably rhenium, alkali metal and silver are in the same impregnating solution, although it is believed that their presence in different solutions will still provide suitable catalysts. The preferred amount of rhenium, calculated as the metal, deposited on or present on the carrier or catalyst ranges from 0.01 mmoles to 15 mmoles, more preferably from 0.2 mmoles to 5 mmoles and most preferably from 0.3 to 3.5 mmoles per kilogram of total catalyst. The degree of benefit obtained within the above-defined limits will vary depending upon particular properties and characteristics, such as, for example, reaction conditions, catalyst preparative conditions, surface area and pore structure and surface chemical properties of the carrier utilized, silver and alkali or other further metal content of the catalyst, and other compounds, anions or cations present beside those containing rhenium or alkali metal, such as the ions added with the alkali metal or rhenium, or compounds remaining from the impregnation technique, and the above-defined limits were selected to cover the widest possible variations in properties and characteristics. These variations are readily determined by experimentation. For purposes of convenience, the amount of rhenium present on the catalyst is measured as the metal, irrespective of the form in which it is present.

The promoting effect provided by the rhenium can be affected by a number of variables such as, for example, reaction conditions, catalyst preparative techniques, surface area and pore structure and surface chemical properties of the support, the silver and further metal content of the catalyst, the presence of other compounds, cations and anions present on the catalyst alone or in combination with the alkali metal and/or rhenium such as the ions added with the alkali metal or rhenium, or compounds remaining from the impregnating solution. The presence of other activators, stabilizers, promoters, enhancers or other catalyst improvers can also affect the promoting effects of the rhenium. It is understood that any supported silver-based, alkali metal promoted ethylene oxide catalyst which contains other cations and/or anions or any other activators, promoters, enhancers, stabilizers or other catalyst improvers and which contains an amount of rhenium which provides a promoting effect, more preferably which provides higher ethylene oxidation selectivities to ethylene oxide at a given oxygen conversion level and most preferably which provides higher initial ethylene oxidation selectivities than is obtained under the same reaction conditions with the same catalyst not containing a promoting amount of rhenium will fall within the scope of the instant invention and claims.

The rhenium compounds, salts and/or complexes used in the preparation of the instant catalysts are

7

rhenium compounds, salts, and/or complexes that can be solublized in an appropriate solvent. Preferably the solvent is a water-containing solvent. More preferably the solvent is the same solvent used to deposit the silver and the alkali metal promoter. Examples of rhenium compounds include the rhenium salts such as rhenium halides, the rhenium oxyhalides, the rhenates, the perrhenates, the oxides and the acids of rhenium. A preferred compound to be utilized in the impregnation solution is the perrhenate, preferably ammonium perrhenate. However, the alkali metal perrhenates, alkaline earth metal perrhenates, silver perrhenate, other perrhenates and rhenium heptoxide can also be suitably utilized. Rhenium heptoxide, $Re_2O_7$, when dissolved in water, hydrolyzes to perrhenic acid, $HReO_4$, or hydrogen perrhenate. Thus, for purposes of this specification rhenium heptoxide can be considered to be a perrhenate, i.e., $ReO_4$. It is also understood that there are many rhenium compounds that are not soluble per se in water. However, these compounds can be solubilized by utilizing various acids, bases, peroxides, alcohols, etc. After solubilization these compounds could be used, for example, with an appropriate amount of water or other suitable solvent to impregnate the carrier. Of course, it is also understood that upon solubilization of many of these compounds, the original compound no longer exists after solubilization. For example, rhenium metal is not soluble in water. However, it is soluble in concentrated nitric acid as well as in hydrogen peroxide solution. Thus, by using an appropriate reactive solvent one could use rhenium metal to prepare a solubilized rhenium-containing impregnating solution.

A presently preferred aspect of the instant invention is that the rhenium present on the catalyst is present in a form that is extractable in a dilute aqueous base solution. For the purposes of this specification a 20 millimolar aqueous sodium hydroxide solution was chosen as the standard solution to be used to test the extractability of rhenium on the catalyst. It will be clear to one skilled in the art that other concentrations of sodium hydroxide as well as other bases can be utilized to test the extractability of rhenium. Thus, one skilled in the art can utilize other bases, for example, other alkali metal hydroxides, other alkaline earth metal hydroxides, ammonium hydroxide, organic bases, etc., suitably dissolved in an appropriate solvent to extract rhenium and by comparing it with the 20 millimolar aqueous sodium hydroxide solution used herein can determine whether rhenium extractability with other base solutions will be equivalent to the rhenium extractability with the 20 millimolar sodium hydroxide solution.

In the above-noted presently preferred embodiment, the rhenium is not present in the free metallic state, but rather is present as a compound, complex or ion. In a particularly preferred embodiment, the rhenium on the catalyst is in a form that is extractable by dilute basic solution, and particularly with the 20 millimolar dilute sodium hydroxide solution disclosed herein. The base extraction technique can be used on a fresh catalyst, i.e., a catalyst that has gone through all the appropriate preparative techniques and is ready to be placed in an ethylene oxide reactor, or on a used catalyst, i.e., a catalyst that has been used for the production of ethylene oxide and then removed from the reactor. In a typical test procedure utilized herein a 1 to 10 g sample of fresh or reactor-tested catalyst is extracted with 10 to 50 milliters of the 20 millimolar aqueous sodium hydroxide solution at 100°C for 10 minutes. The amount of rhenium in an aliquot of the cooled extract is determined spectrophotometrically following the procedure of V.W. Meloche et al., Analytical Chemistry, 29, 527 (1957). In this procedure, a coloured rhenium complex with alpha-furildioxime is formed by reduction of the rhenium species with tin (II) chloride in a dilute hydrochloric acid solution containing a large excess of alpha-furildioxime.

Generally, the carrier is contacted with a silver salt, a silver compound, or a silver complex which has been dissolved in an aqueous solution, so that the carrier is impregnated with said aqueous solution, thereafter the impregnated carrier is separated from the aqueous solution, e.g., by centrifugation or filtration and then dried. The thus obtained impregnated carrier is heated to reduce the silver to metallic silver. It is conveniently heated to a temperature in the range from 50°C to 600°C, during a period sufficient to cause reduction of the silver salt, complex or compound to metallic silver and to form a layer of finely divided silver, which is bound to the surface of the carrier, both the exterior and pore surface. Air, or other oxygen containing gas, reducing gas, an inert gas or mixtures thereof may be conducted over the carrier during this heating step.

There are several known methods to add the silver to the carrier or support. The carrier may be impregnated with an aqueous solution containing silver nitrate dissolved therein, and then dried, after which drying step the silver nitrate is reduced with hydrogen or hydrazine. The carrier may also be impregnated with an ammoniacal solution of silver oxalate or silver carbonate, and then dried, after which drying step the silver oxalate or silver carbonate is reduced to metallic silver by heating, e.g., to about 600°C. Specific solutions of silver salts with solubilizing and reducing agents may be employed as well, e.g., combinations of the vicinal alkanolamines, alkylenediamines and ammonia.

One such example of a solution of silver salts comprises an impregnating solution comprising:

A. a silver salt of a carboxylic acid,

B. an organic amine alkaline solubilizing/reducing agent,

C. an aqueous solvent.

Suitable carboxylic acid silver salts include silver carbonate and the silver salts of mono- and polybasic carboxylic and hydroxycarboxylic acids of up to about 16 carbon atoms. Silver carbonate and silver oxalate are particularly useful silver salts, with silver oxalate being most preferred.

An organic amine solubilizing/reducing agent is present in the impregnating solution. Suitable organic amine silver solubilizing/reducing agents include lower alkylenediamines of from 1 to 5 carbon atoms, mixtures of a lower alkanolamine of from 1 to 5 carbon atoms with a lower alkylenediamine of from 1 to 5 carbon atoms, as well as mixtures of ammonia with lower alkanolamines or lower alkylenediamines of from 1 to 5 carbons. Four groups of organic amine solubilizing/reducing agents are particularly useful. They are the following:

A. vicinal alkylenediamines of from 2 to 4 carbon atoms;

B. mixtures of (1) vicinal alkanolamines of from 2 to 4 carbon atoms and (2) vicinal alkylenediamines of from 2 to 4 carbon atoms;

C. mixtures of vicinal alkylenediamines of from 2 to 4 carbon atoms and ammonia; and

D. mixtures of vicinal alkanolamines of from 2 to 4 carbon atoms and ammonia. These solubilizing/reducing agents are generally added in the amount of from 0.1 to 10 moles per mole of silver present.

Particularly preferred solubilizing/reducing agents are:

A. ethylenediamine,

B. ethylenediamine in combination with ethanolamine,

C. ethylenediamine in combination with ammonia, and

D. ethanolamine in combination with ammonia.

Ethylenediamine is most preferred. Ethylenediamine in combination with ethanolamine gives comparable results, but it is believed that impurities that are present in certain commercially available ethanolamine preparations can produce inconsistent results.

When ethylenediamine is used as the sole solubilizing/reducing agent, it is necessary to add amounts of the amine in the range of from 0.1 to 5.0 moles of ethylenediamine per mole of silver.

When ethylenediamine and ethanolamine together are used as the solubilizing/reducing agent, it is suitable to employ from 0.1 to 3.0 moles of ethylenediamine per mole of silver and from 0.1 to 2.0 moles of ethanolamine per mole of silver.

When ethylenediamine or ethanolamine is used with ammonia, it is generally useful to add at least about two moles of ammonia per mole of silver and very suitable to add from about 2 to about 10 moles of ammonia per mole of silver. The amount of ethylenediamine or ethanolamine employed then is suitably from 0.1 to 2.0 moles per mole of silver.

One method of preparing the silver-containing catalyst can be found in US-A 3,702,259 incorporated by reference herein. Other methods for preparing the silver-containing catalysts can be found in US-A 4,010,115; 4,356,312; 3,962,136 and US-A 4,012,425, all incorporated by reference herein.

The preferred amount of alkali or further metal promoter deposited on or present on the surface of the carrier or catalyst generally lies between 10 and 3000, preferably between 15 and 2000 and more preferably between 20 and 1500 ppm by weight of alkali metal calculated on the total catalyst material. Amounts between 50 and 1000 ppm are most preferable. Suitable compounds of alkali metal or further metal are, for example, the nitrates, carbonates, bicarbonates, oxalates, carboxylic acid salts or hydroxides put in solution, preferably aqueous solution. The most preferred alkali promoter is cesium, preferably applied as an aqueous solution having cesium nitrate or cesium hydroxide dissolved therein. While the higher alkali metals provide the most significant effect when considering the selectivity, particularly the initial selectivity, it is considered within the scope of the instant preferred embodiment that lithium and/or sodium may also be present in addition to the higher alkali metal(s) in order to provide enhanced or different effects.

There are known excellent methods of applying the promoters coincidentally with the silver on the carrier. Suitable alkali metal salts are generally those which are soluble in the silver-impregnating liquid phase. Besides the above-mentioned compounds may be mentioned the nitrites; the halides, such as fluorides, chlorides, iodides, bromides; oxyhalides; bicarbonates; borates; sulfates; sulfites; bisulfates; acetates; tartrates; lactates and isopropoxides, etc. The use of rhenium or alkali metal salts which have ions which react with the silver ions in solution is preferably avoided, e.g. the use of cesium chloride together with silver nitrate in an aqueous solution, since then some silver chloride is prematurely precipitated. Here the use of cesium nitrate is recommended instead of cesium chloride, for example. However, cesium chloride may be used together with a silver salt-amine-complex in aqueous solution, since then the silver chloride is not precipitated prematurely from the solution.

The promoters may be deposited on the carrier (support) or on the catalyst depending upon the particular impregnation technique or sequence utilized. As used in this specification and claims, the term "on the catalyst" when referring to the deposition or presence of promoters and/or co-promoters refers to the catalyst which comprises the combination of carrier (support) and silver. Thus, the promoters, i.e., alkali metal and rhenium may be found individually or in a mixture thereof on the catalyst, on the support or on both the catalyst and the support. There may be, for example, alkali and rhenium on the catalyst; alkali and rhenium on the support; alkali on the support and rhenium on the catalyst; alkali on the support and a mixture of alkali and rhenium on the catalyst; rhenium on the support and a mixture of alkali and rhenium on the catalyst; rhenium on the support and alkali on the catalyst; a mixture of alkali and rhenium on the support and a mixture of alkali and rhenium on the catalyst; a mixture of alkali and rhenium on the support and alkali on the catalyst; and a mixture of alkali and rhenium on the support and rhenium on the catalyst.

The amount of the alkali metal and/or rhenium promoters on the porous carrier or catalyst may also be regulated within certain limits by washing out the surplus of promoter material with an appropriate solvent, for example, methanol or ethanol.

A particularly preferred process of impregnating the carrier consists of impregnating the carrier with an aqueous solution containing a silver salt of a carboxylic acid, an organic amine, a salt of cesium and ammonium perrhenate dissolved therein. Silver oxalate is a preferred silver salt. It can be prepared by reacting silver oxide (slurry in water) with (a) a mixture of ethylenediamine and oxalic acid, or (b) oxalic acid and then ethylenediamine, which latter is preferred, so that an aqueous solution of silver oxalateethylenediamine complex is obtained, to which solution is added a certain amount of cesium compound and ammonium perrhenate. While addition of the amine to the silver oxide before adding the oxalic acid is possible, it is not preferred since it can give rise to solutions which are unstable or even explosive in nature. Other diamines and other amines, such as ethanolamine, may be added as well. A cesium-containing silver oxalate solution may also be prepared by precipitating silver oxalate from a solution of cesium oxalate and silver nitrate and rinsing with water or alcohol the obtained silver oxalate in order to remove the adhering cesium salt until the desired cesium content is obtained. The cesium-containing silver oxalate is then solubilized with ammonia and/or an amine in water and ammonium perrhenate is added. Rubidium-, potassium-, sodium-, lithium- and mixtures of alkali metal-containing solutions may be prepared also in these ways. The impregnated carriers are then heated to a temperature between $50°C$ and $600°C$, preferably between $75°C$ and $400°C$ to evaporate the liquid and produce a metallic silver.

In general terms, the impregnation process comprises impregnating the support with one or more solutions comprising silver, further metal and rhenium. As used in the instant specification and claims, the terminology "impregnating the support with one or more solutions comprising silver, further metal and/or rhenium" and similar or cognate terminology means that the support is impregnated in a single or multiple impregnation, with one solution containing silver, further metal, e.g. alkali metal and rhenium; in multiple impregnations with two or more solutions containing silver, further metal and rhenium in differing amounts; or in multiple impregnations with two or more solutions, wherein each solution contains at least one component selected from silver, further metal and rhenium with the proviso that all of the components silver, further metal and rhenium will individually be found in at least one of the solutions. The concentration of the silver (measured as the metal) in the silver-containing solution will range from 1 g/liter up to the solubility limit of silver in the solution, preferably from 10 g/l up to the solubility limit when a single impregnation is utilized. The concentration of the further metal (measured as the metal) will range from $1 \times 10^{-3}$ g/liter up to 12 g/liter, preferably from $10 \times 10^{-3}$ g/l to 12 g/l when a single impregnation is utilized. The concentration of the rhenium (measured as the metal) will range from about $5 \times 10^{-3}$ g/l to about 20 g/l, preferably from about $50 \times 10^{-3}$ g/l to about 20 g/l when a single impregnation step is utilized. Concentrations selected within the above-noted ranges will depend upon the pore volume of the catalyst, the final amount desired in the final catalyst and whether the impregnation is single or multiple. Appropriate concentrations can readily be determined by routine experimentation.

It is observed that independent of the form in which the silver is present in the solution before precipitation on the carrier, the term "reduction to metallic silver" is used, while in the meantime often decomposition by heating occurs. It is preferred to use the term "reduction", since the positively charged $Ag^+$ ion is converted into metallic Ag atom. Reduction times may generally vary from about 0.5 minute to about 8 hours, depending on the circumstances.

The silver catalysts according to the present invention have been shown to have a particularly high initial selectivity for ethylene oxide in the direct oxidation of ethylene with molecular oxygen to ethylene oxide. The conditions for carrying out such an oxidation reaction in the p resence of the silver catalysts according to the present invention broadly comprise those already described in the prior art. This applies, for example, to suitable temperatures, pressures, residence times, diluent materials, such as nitrogen, carbon dioxide, steam, argon, methane or other saturated hydrocarbons, presence of moderating agents to control the catalytic action, for example, 1-2-dichloroethane, vinyl chloride or chlorinated polyphenyl compounds, the desirability of employing recycle operations or applying successive conversions in different reactors to increase the yields of ethylene oxide, and any other special conditions which may be selected in processes for preparing ethylene oxide. Pressures in the range of from atmospheric to 35 bar are generally employed. Higher pressures are, however, by no means excluded. Molecular oxygen employed as reactant can be obtained from conventional sources. The suitable oxygen charge may consist essentially of relatively pure oxygen, a concentrated oxygen stream comprising oxygen in major amount with lesser amounts of one or more diluents, such as nitrogen and argon, or another oxygencontaining stream, such as air. It is therefore evident that the use of the present silver catalysts in ethylene oxidation reactions is in no way limited to the use of specific conditions among those which are known to be effective. For purposes of illustration only, table 2 shows the range of conditions that are often used in current commercial ethylene oxide reactor units.

### TABLE 2

| | |
|---|---|
| * GHSV | 1500 - 10,000 |
| Inlet pressure, $kg \cdot cm^{-2}$ | ~~150~~ - 400 ~~psig~~ |
| | 10.5      38 |
| Inlet Feed | |
| ethylene | 1 - 40% |
| $O_2$ | 3 - 12% |
| $CO_2$ | 2 - 40% |
| ethane | 0 - 3% |
| Argon and/or methane and/or nitrogen diluent | |
| chlorohydrocarbon moderator | 0.3 - 20 ppmv total |
| Coolant temperature | 180 - 315°C |
| Catalyst temperature | 180 - 325°C |
| $O_2$ conversion level | 10 - 60% |
| EO Production (Work Rate) | 32 - 256 $kg$. EO $m^{-3}$ catalyst $hr^{-1}$ |

* Liters of gas at standard temperature and pressure passing over the one liter of packed catalyst per hour.

In a preferred application of the silver catalysts according to the present invention, ethylene oxide is produced when an oxygen-containing gas is contacted with ethylene in the presence of the present catalysts at a temperature in the range of from 180 to 330° C and preferably 200 to 325° C.

In a further preferred embodiment the catalysts, in addition to first (rhenium) and second (further metal) promoter, comprise sulphur as a further promoting (copromoting) additive.

The exact form of the co-promoter on the catalyst is not known. The co-promoter, it is believed, is not present on the catalyst in the elemental form since the co-promoter is applied to the catalyst in the form of ions, salts, compounds and/or complexes and the reducing conditions generally used to reduce the silver to metallic silver are not usually sufficient to reduce the sulphur to the elemental form. It is believed that the co-promoter deposited on the support or present on the catalyst is in the compound form, most probably in the form of an oxygen-containing compound. In a presently preferred embodiment, the co-promoter is applied to the catalyst in the oxyanionic form, i.e., in the form of an anion, or negative ion which contains oxygen. Examples of anions of sulphur that can be suitably applied include sulfate, sulfite, bisulfite,

11

bisulfate, sulfonate, persulfate, thiosulfate, dithionate, dithionite, etc. Preferred compounds to be applied are ammonium sulfate and the alkali metal sulfates. Compounds of molybdenum, tungsten and chromium can also be used as co-promoter as set out hereinbefore. Suitable compounds are molybdate, dimolybdate, paramolybdate, other iso- and hetero-polymolybdates, etc.; tungstate, paratungstate, metatungstate, other iso- and hetero-polytungstates, etc.; and chromate, dichromate, chromite, halochromate, etc. Preferred are sulfates, molybdates, tungstates and chromates. The anions can be supplied with various counter-ions. Preferred are ammonium, alkali metal and hydrogen (i.e. acid form). The anions can be prepared by the reactive dissolution of various non-anionic materials such as the oxides such as $SO_2$, $SO_3$, $MoO_3$, $WO_3$, $Cr_2O_3$, etc., as well as other materials such as halides, oxyhalides, hydroxyhalides, hydroxides, sulfides, etc., of the metals.

The carrier is impregnated with rhenium co-promoter ions, salt(s), compound(s) and/or complex(es). This may be done at the same time that the other components are added or before and/or later. Preferably sulphur, molybdenum, tungsten or chromium co-promoter, rhenium, alkali metal and silver are in the same impregnating solution, although it is believed that their presence in different solutions will still provide suitable catalysts.

The preferred amount of sulphur co-promoter compound present on or deposited on the support or catalyst ranges from 0.1 to 10 mmoles, preferably from 0.2 to 5 millimoles, measured as the element, per kilogram of total catalyst. Preferred co-promoter compounds are the oxyanionic compounds of the co-promoter elements, preferably the ammonium and alkali metal oxyanionates, such as ammonium sulfate, potassium sulfate, cesium chromate, rubidium tungstate, ammonium molybdate, lithium sulfate, sodium tungstate, lithium chromate and the like.

A process for producing ethylene oxide by catalytically reacting ethylene and oxygen in vapour phase in the presence of a catalyst composition comprising silver, a support, a promoting amount of rhenium compound thereof and a promoting amount of at least one further metal or compound thereof is also included within the scope of the invention. In this process the preferred catalysts are those comprising a support having a surface area of less than 20 m²/g.

In a further aspect this invention is concerned with an ethylene oxide catalyst comprising a porous support, from 1 to 30 %wt of silver, from 0.01 to 15 mmol/kg cat. of rhenium and from 10 to 3000 ppm of further metal or metal compound. In the latter catalysts, preferred supports, preferred pore volume and surface area of support, preferred proportions of rhenium promoter, of further metal promoter, optionally of sulphur co-promoter and of silver are as set out hereinbefore. Within the latter group of catalysts preference is given to those catalysts that are able to perform at a higher selectivity at a given oxygen conversion level than is obtained under the same reaction conditions within the same combination of silver, and support, none or one of the promoters selected from rhenium and further metal or metal compound. Even more preferred in this group are catalysts that are able to perform at a higher selectivity at a given oxygen conversion level than is obtained under the same reaction conditions with the same combination of silver, support and further metal promoter but no rhenium.

The reaction conditions for testing the selectivity performance are preferably those set out in Example 1.

## EXAMPLE 1

The following illustrative embodiment describes typical preparative techniques for making the catalysts of the instant invention (and comparative catalysts) and the typical technique for measuring the properties of these catalysts.

Part A: Preparation of stock silver oxalate/ethylenediamine solution for use in catalyst preparation:

1) Dissolve 415g reagent-grade NaOH in 2340 ml deionized water. Adjust temperature to 50°C.

2) Dissolve 1699 g "spectropure" (high-purity) $AgNO_3$ in 2100 ml deionized water. Adjust temperature to 50°C.

3) Add NaOH solution slowly to $AgNO_3$ solution with stirring, maintaining temperature at 50°C. Stir for 15 minutes after addition is complete, then lower temperature to 40°C.

4) Insert clean filter wands and withdraw as much water as possible from the precipitate created in step (3) in order to remove sodium and nitrate ions. Measure the conductivity of the water removed and add back as much fresh deionized water as was removed by the filter wands. Stir for 15 minutes at 40°C. Repeat this process until the conductivity of the water removed is less than 90 μohm/cm. Then add back 1500 ml deionized water.

12

5) Add 630g of high-purity oxalic acid dihydrate in approximately 100g increments. Keep the temperature at 40° C and stir to mix thoroughly. Add the last portion of oxalic acid dihydrate slowly and monitor pH to ensure that pH does not drop below 7.8. Aim for a pH endpoint of 8.0-8.4. Add high-purity silver oxide if necessary to achieve this endpoint.

6) Remove as much water from the mixture as possible using clean filter wands in order to form a highly concentrated silver-containing slurry. Cool the silver oxalate slurry to 30° C.

7) Add 699g of 92%w ethylenediamine (8% deonized water). Do not allow the temperature to exceed 30° C during addition.

The above procedure yields a solution containing approximately 27-33 %w Ag.

### Part B: Catalyst Impregnation Procedures

Catalyst support Example B described in Table 1 is a preferred support for the instant invention and is used in the following examples and illustrative embodiments unless otherwise stated.

Preparation of undoped impregnating solution is as follows: The stock Ag oxalate/ethylenediamine solution of Part A is diluted preferably with deionized water, or alternatively may be diluted with monoethanolamine, or a mixture of deionized water and monoethanolamine to achieve a Ag concentration of approximately 27.6% by weight. The use of monoethanolamine or monoethanolamine plus water to dilute the stock solution is believed to provide catalysts comparable to those obtained by the use of water. However, it is believed that certain impurities present in monoethanolamine can cause inconsistent results in the catalysts made with monoethanolamine. Hence, water is preferred and was used for all of the examples provided herein.

Preparation of doped impregnation solution is as follows:

For catalyst A (Cs only): Add 46.4 mg of aqueous CsOH solution (50.7% w Cs) directly to 50g of undoped impregnating solution.

For catalyst B (Cs - Re): Dissolve 55.0 mg of $NH_4ReO_4$ in a minimum volume of 50/50 (w/w) ethylenediamine/deionized water and add to 50g of undoped impregnating solution. Then add 84.7 mg of aqueous CsOH solution (50.7 %w Cs) to the same impregnating solution.

For catalyst C(Cs-Re-S): Dissolve 27.4 mg of $NH_4ReO_4$ plus 13.5 mg of $(NH_4)SO_4$ in a minimum volume of 50/50 (w/w) ethylenediamine/deionized water and add to 50g of undoped impregnating solution. Then add 82.7 mg of aqueous CsOH solution (50.7 %wt Cs) to the same impregnating solution.

The aqueous cesium hydroxide solution used for catalyst preparation in this and the following illustrative embodiments was doped with a radioactive isotope of cesium ([134]Cs) so that the cesium levels on the finished catalysts could be readily determined by radiotracer analysis. (Alternatively, the levels of cesium and other alkali promoters on finished catalysts can be determined by the water leaching method described below.) The concentration of cesium in this aqueous, radiolabeled cesium hydroxide solution was determined to be 50.7 w% by neutron activation analysis at the Nuclear Science Center, Texas A&M University, College Station, Texas, using a TRIGA reactor, an Ortec high-purity Germanium Model BA-GEM-25185 detector, and a Tracor Northern Model 4000 multichannel analyzer. All target and actual cesium levels reported for catalysts in this and the following Illustrative Embodiments are based upon a value of 50.7 %w for the concentration of cesium in the stock, radiolabeled cesium hydroxide solution. However, when this same cesium hydroxide solution was subsequently analyzed by inductively coupled plasma jet-mass spectrometry using a SCIEX Elan 250 instrument, the cesium concentration was found to be 45 %w. If this latter value for the cesium concentration in this solution is closer to the actual value, then the absolute levels of cesium for the catalysts described in this and the following Illustrative Embodiments would be approximately 11.2% lower than those reported.

### Part C: Catalyst impregnation and curing

Approximately 30g of carrier B are placed under 25 mm vacuum for 3 minutes at room temperature. Approximately 50g of doped impregnating solution is then introduced to submerge the carrier, and the vacuum is maintained at 25 mm for an additional 3 minutes. At the end of this time, the vacuum is released, and excess impregnating solution is removed from the carrier by centrifugation for 2 minutes at 500 rpm. If the impregnating solution is prepared without monoethanolamine, then the impregnated carrier is then cured by being continuously shaken in a 8.5m³/hr (300 cu. ft./hr.) air stream flowing across a cross-sectional area of approximately (3-5 square inches) at 250° C for 5 minutes. If $1.93$-$3.22 \times 10^{-3}$ m² significant mon-oethanolamine is present in the impregnating solution, then the impregnated carrier is cured by being continuously shaken in a 300 cu. ft./hr. air stream at 250° C for 2.5 minutes, followed by a 2.83m³/hr (100 cu. ft./hr.) air stream at 270°C for 7.5 minutes (all over a cross-section area of approximately $1.93 \times 10^{-3} m^2 - 3.22 \times 10^{-3} m^2$ 3-5 square inches)). The cured catalyst is then ready for testing.

This procedure will yield catalysts on this carrier containing approximately 13.5% w Ag with the following approximate dopant levels and which are approximately optimum in cesium for the given silver and rhenium and sulphur levels and support with regard to initial selectivity under the test conditions described below.

|  | Cs, ppmw | Re, ppmw | S, ppm |
|---|---|---|---|
| catalyst A | 230 | 0 | 0 |
| B | 420 | 372 | 0 |
| C | 410 | 186 | 32 |

The actual silver content of the catalyst can be determined by any of a number of standard, published procedures. The actual level of rhenium on the catalysts prepared by the above process can be determined by extraction with 20 mM aqueous sodium hydroxide, followed by spectrophotometric determination of the rhenium in the extract, as described above. The actual level of cesium on the catalyst can be determined by employing a stock cesium hydroxide solution, which has been labeled with a radioactive isotope of cesium, in catalyst preparation. The cesium content of the catalyst can then be determined by measuring the radioactivity of the catalyst. Alternatively, the cesium content of the catalyst can be determined by leaching the catalyst with boiling deionized water. In this extraction process cesium, as well as other alkali metals, is measured by extraction from the catalyst by boiling 10 grams of whole catalyst in 20 milliliters of water for 5 minutes, repeating the above two more times, combining the above extractions and determining the amount of alkali metal present by comparison to standard solutions of reference alkali metals using atomic absorption spectroscopy (using Varian Techtron Model 1200 or equivalent).

Part D: Standard Microreactor Catalyst Test Conditions/Procedure

3 to 5g of crushed catalyst (14-20 mesh) are loaded into a $\frac{1}{4}$ inch diameter stainless steel U-shaped tube. The U tube is immersed in a molten metal bath (heat medium) and the ends are connected to a gas flow system. The weight of catalyst used and the inlet gas flow rate are adjusted to achieve a gas hourly space velocity of 3300 cc of gas per cc of catalyst per hour. The inlet gas pressure is 210 psig.

The gas mixture passed through the catalyst bed (in once-through operation) during the entire test run (including startup) consists of 30% ethylene, 8.5% oxygen, 7% carbon dioxide, 54.5% nitrogen, and 4.4 to 5.6 ppmv vinyl chloride.

The initial reactor (heat medium) temperature is 180°C. After 1 hour at this initial temperature, the temperature is increased to 190°C for 1 hour, followed by 200°C (1 hour), 210°C (1 hour), 220°C (1 hour), 227°C (2 hours), 235°C (2 hours), and 242°C (2 hours). The temperature is then adjusted so as to achieve a constant oxygen conversion level of 40%. Performance data at this conversion level are usually obtained when the catalyst has been onstream for a total of 16 ± 4 hours and are referred to as "initial performance data" in the examples given below. Due to slight differences in feed gas composition, gas flow rates, and the calibration of analytical instruments used to determine the feed and product gas compositions, the measured selectivity and activity of a given catalyst may vary slightly from one test run to the next. To allow meaningful comparison of the performances of catalysts tested at different times, all catalysts described in this and the following illustrative embodiments were tested simultaneously with a standard catalyst having the composition of catalyst A or with a different catalyst which has been standardized with reference to catalyst A. All performance data reported in this and the following illustrative embodiments are corrected and stated relative to the average initial performance of catalyst A ($S_{40}$ = 80.0%; $T_{40}$ = 242°C).

Typical initial performances at 40% $O_2$ conversion for the above recipes are as follows:

catalyst A selectivity = 80.0% temperature = 242°C
catalyst B selectivity = 81.9% temperature = 248°C
catalyst C selectivity = 82.9% temperature = 253°C

Example 2

Using the general preparative technique of Example 1, a series of catalysts were prepared utilizing carrier B described in Table 1. The catalysts were prepared without using monoethanolamine. One series of catalysts contained 2 mmol (millimoles) of rhenium per kilogram of catalyst, the second series contained 1 mmol of rhenium and 1 mmol of sulphur per kilogram of catalyst and the third series of catalysts was made in the identical fashion except that they contained no rhenium or no sulphur. In all three series the concentration of cesium in the individual catalysts was varied. The catalysts were tested as described in Example 1 and the results are shown in Table 3. The cesium levels reported in Table 3 were obtained by the radiotracer analysis technique described in Example 1, assuming a concentration of 50.7 %w cesium for the radiolabeled, aqueous cesium hydroxide solution used in catalyst preparation. Further, the results from these tests in the form of the initial selectivity versus cesium concentration are plotted in Figure 1. In this Figure one can see the beneficial effects of rhenium which are indicated by the highlighted area between the two curves to the right of their cross-over point. It can be seen from Figure 1 that the use of rhenium provides not only an increase in the absolute value of the initial selectivity obtained at optimum cesium concentration, but also a significantly improved initial selectivity of the catalyst at high cesium concentrations e.g., 300 ppm cesium and over. In figure 2, likewise initial selectivity versus cesium concentration is plotted. In this Figure 2 one can see the beneficial effects of rhenium plus sulphur which are indicated by the highlighted area between the two curves A and C to the right of their crossover point. It can be seen from the Figure that the use of rhenium plus sulphur provides not only an increase in the absolute value of the initial selectivity obtained at optimum cesium concentration, but also a significantly improved initial selectivity of the catalyst at high cesium concentrations e.g., 300 ppm cesium and over when compared to catalysts containing no rhenium. The addition of the sulphur co-promoter also provides a higher initial selectivity over the case where no co-promoter is used.

EXAMPLE 3

A series of catalysts were prepared in a fashion similar to the technique described in Example 1 using different carriers having those properties described in Table 1 in the specification. The catalysts were made without monoethanolamine. The catalysts were tested as described in Example 1 and the results are shown below in Table 4. Unless otherwise noted, all catalysts listed in Table 4 have cesium levels which give the optimum (highest) initial selectivity obtained under these conditions for a catalyst made on the indicated carrier with the indicated levels of silver and rhenium. The cesium levels reported in Table 4 were obtained by the radiotracer analysis technique described in Example 1, assuming a concentration of 50.7 %w cesium for the radiolabeled, aqueous cesium hydroxide solution used in catalyst preparation. Catalyst 4-6 was not made using the identical support of catalyst 4-5 but rather used a comparable support from a different lot which had a surface area of 0.44 $m^2$/g, a water pore volume of 0.42 ml/g, a water-leachable sodium content approximately 50% higher and an acid-leachable sodium content approximately 100% higher. (This support is referred to hereinafter as C').

EXAMPLE 4

A series of catalysts were prepared in a fashion similar to that described in Example 1 utilizing the support described in Example 2, but utilizing different rhenium and sulphur concentrations. The catalysts were tested as described in Example 1 and the results are shown in Table 5 below. Unless otherwise noted, all catalysts listed in Table 5 have cesium levels which give the optimum (highest) initial selectivity obtained under these test conditions for a catalyst made on this support with the indicated levels of silver, rhenium and sulphur. The cesium levels reported in Table 5 were obtained by the radiotracer analysis technique described in Example 1, assuming a concentration of 50.7 %w cesium for the radiolabeled, aqueous cesium hydroxide solution used in catalyst preparation.

EXAMPLE 5

A series of catalysts were prepared in a fashion similar to that described in Example 1 using the support described in Example 2. The catalysts were made without monoethanolamine. In this series different alkali metals were utilized as alkali metal hydroxides. The catalysts were tested as described in Example 1 and the results are shown in Table 6 below. Unless otherwise noted, all catalysts listed in Table 6 have alkali levels which give the optimum (highest) initial selectivity obtained under these test conditions for a catalyst made with the indicated alkali metal hydroxide on this support with the indicated levels of silver, rhenium and sulphur. The alkali levels presented represent target levels.

For runs 6-19 and 6-20, the target cesium content was fixed at 160 ppm and the rubidium concentration was optimized to provide the highest initial selectivity under these test conditions at the indicated levels of silver and rhenium. Also, for these two examples, the support, which was otherwise comparable to support B, had a surface of 0.45 m$^2$/g instead of 0.42 m$^2$/g and about 10-15% lower levels of leachable sodium (this support is referred to hereinafter as support B').

## EXAMPLE 6

Two sets of catalysts were prepared in a fashion similar to that described in Example 1 using support Example B of Table 1 with the exception that ammonium molybdate ($(NH_4)_6$ $Mo_7O_{24}.4H_2O$) was added to the impregnation solution in sufficient quantity to provide about 96 ppm by weight of Mo in the final catalyst. The catalysts were made without monoethanolamine. The catalysts contained potassium (target) levels which provide the optimum (highest) initial selectivity under the test conditions described in Example 1 at the noted levels of silver, rhenium and molybdenum. Catalyst example VI-1 (prepared using support B') which contained 13.2 %w silver, no rhenium, 180 ppm K (target level) and 96 ppm Mo had an initial $S_{40}$ of 77.0% and an initial $T_{40}$ of 261 °C and catalyst example VI-2 which contained 14.5 %w silver, 186 ppm by weight of rhenium (target level), 160 ppm K (target level) and 96 ppm Mo (target level) had an initial $S_{40}$ of 81.1% and an initial $T_{40}$ of 279 °C. For comparative purposes, a catalyst not containing rhenium or molybdenum but otherwise having the same composition, has a $S_{40}$ of 79.4 and a $T_{40}$ of 240 °C.

## EXAMPLE 7

Two catalysts were prepared in a fashion similar to that described in Example 1 using support Example B of Table 1. The catalysts both contained cesium levels which had been optimized to provide the highest initial selectivities under the test conditions described in Example 1. Both catalysts were made without using monoethanolamine.

Catalyst VII-1 was prepared using 2 $\mu$moles/g each of $NH_4ReO_4$ and $(NH_4)_2SO_4$. Catalyst VII-2 was prepared using 2 $\mu$moles/g each of $(NH_4)ReO_4$ and $Na_2SO_4$. The catalysts were tested as described in Example 1 and the results are listed below:

| Catalyst | %w Ag | Cs* ppmw | Na** ppmw | Re** $\mu$moles/g | $S_{40}$ | $T_{40}$ |
|---|---|---|---|---|---|---|
| VII-1 | 12.8 | 513 | 0 | 2 | 81.7% | 274°C |
| VII-2 | 13.5 | 424 | 92 | 2 | 83.9% | 253°C |

\* by radiotracer, assuming a concentration of 50.7 %w cesium for the radiolabeled, aqueous cesium hydroxide solution used in catalyst preparation.

\*\* target levels

It can be seen from the above results that the catalyst containing the mixture of cesium and sodium as alkali metal promoters is more selective and more active than the catalyst which contains only cesium as the alkali metal promoter.

## EXAMPLE 8

Three catalysts were prepared in a fashion similar to that described in Example 1 (no monoethanolamine) using support Example B of Table 1. The catalysts contained cesium levels which had been optimized to provide the highest initial selectivities under the test conditions described in Example 1. Catalyst VIII-1 was prepared using one $\mu$mole/g of $NH_4ReO_4$ and two $\mu$moles/g of $(NH_4)_2SO_4$. Catalyst VIII-2 was prepared using one $\mu$mole/g of $NH_4ReO_4$ and two $\mu$moles/g of $Li_2SO_4$. Catalyst VIII-3 was prepared using one $\mu$mole/g of $NH_4ReO_4$ and two $\mu$moles/g of $Na_2SO_4$. The catalysts were tested as described in Example 1 and the results are listed below:

| Catalyst | %w Ag | Cs* ppmw | Li* ppmw | Na* ppmw | Re $\mu$moles/g | $S_{40}$ | $T_{40}$ |
|---|---|---|---|---|---|---|---|
| VIII-1 | 13.8 | 505 | 0 | 0 | 1 | 82.0% | 273°C |
| VIII-2 | 13.9 | 398 | 28 | 0 | 1 | 83.1% | 249°C |
| VIII-3 | 14.8 | 411 | 0 | 92 | 1 | 80.9% | 248°C |

* by radiotracer, assuming a concentration of 50.7 %w cesium for the radiolabeled, aqueous cesium hydroxide solution used in catalyst preparation.

** target levels

It can be seen from the above results that Catalyst VIII-2 containing a mixture of cesium plus lithium as the alkali promoters is more selective and more active than the catalyst with only cesium as the alkali promoter. At this level of rhenium (half of that of Example 7), Catalyst VIII-3 containing both cesium plus sodium as alkali promoters shows improved activity over the comparable catalyst containing only cesium as alkali promoter whereas the selectivity is diminished.

EXAMPLE 9

A series of catalysts were prepared in a fashion similar to that described in Example 1 using the support described in Example 2. In this series different combinations of alkali promoter(s), rhenium and rhenium co-promoter(s) were utilized. The catalysts were tested as described in Example 1 and the results are shown in Table 7 below.

Unless otherwise noted, all catalysts listed in Table 7 have cesium (or other alkali) levels which give the optimum (highest) initial selectivity obtained under these test conditions for a catalyst made on this support with the indicated levels of silver, rhenium, and rhenium co-promoter(s) and (if added) other alkali(s). The cesium levels reported in Table 7 were obtained by the radiotracer analysis technique described in Example 1, assuming a concentration of 50.7 %w cesium for the radiolabeled, aqueous cesium hydroxide solution used in catalyst preparation. The levels of the other alkalis given in Table 7 represent target levels. Catalyst 7-31 utilized a support which was comparable to Example B but which had a surface area of 0.45 m²/g instead of 0.42 m²/g and had about 10-15% lower levels of leachable sodium.

EXAMPLE 10

A catalyst was prepared by impregnating a carrier similar to carrier B in Table 1 with a solution comprising silver ions, cesium ions, rhenium-containing ions and sulphur-containing ions similar to the method described in Example 1, Part C. The catalyst contained approximately 13.5% silver, 500 ppm cesium, (measured by radiotracer analysis), 260 ppm rhenium and 35 ppm S. This catalyst was tested over a period of about 2 months in a U-tube reactor under operating conditions similar to that described in Part D of Example 1. The results (maximum selectivities and corresponding activities measured as coolant temperature, both measured at an oxygen conversion of 40%) as shown in Table 8.

Table 8

| Time (Days) | Selectivity, % | Coolant Temperature, °C |
|---|---|---|
| 1 | 86.1 | 249 |
| 8 | 86.1 | 250 |
| 20 | 86.5 | 251 |
| 30 | 86.4 | 252 |
| 39 | 86.2 | 253 |
| 47 | 86.6 | 254 |
| 59 | 86.1 | 256 |

EXAMPLE 11

Carrier D was impregnated with barium acetate to provide 2 mmoles/kg of barium on the carrier. Subsequently the impregnated carrier was dried and calcined at about 800°C for 3 hours. This barium-treated carrier was used to prepare a catalyst by a method similar to that described in Example 1. The catalyst contained about 14.8% Ag; 2 mmoles/kg Ba; 1 mmole/kg Re, 1 mmole/kg S, and 549 ppm Cs (radiotracer analysis). A comparison catalyst was prepared using Carrier D without any barium treatment. This catalyst contained about 14.5% Ag; 1 mmole/kg Re; 1 mmole/kg S and 570 ppm Cs (radiotracer analysis). These catalysts were tested for ethylene oxide synthesis by a process similar to that described in Part D of Example 1. While the non-barium-containing catalyst had a higher initial selectivity at 40% oxygen conversion to ethylene oxide than the barium-containing catalyst, at 4-5 days at 40% oxygen conversion, the barium-containing catalyst exhibited a selectivity of about 0.5% greater than the non-barium containing catalyst.

TABLE 3 - CS OPTIMIZATION ON CS ONLY; CS/RE; AND CS/RE+S CATALYSTS

| Experiment No. | %wAg | Re Target Level, ppmw | S Target Level, ppmw | Cs, ppmw (Radiotracer Analysis) | Initial $S_{40}$, % | Initial $T_{40}$, °C |
|---|---|---|---|---|---|---|
| 3-1*** | 13.6 | 0 | 0 | 0 | 74.6 | 229 |
| 3-2*** | 13.6 | 0 | 0 | 104 | 77.6 | 232 |
| 3-3 | 14.3 | 0 | 0 | 236 | 80.0 | 242 |
| 3-4 | 14.3 | 0 | 0 | 301 | 79.4 | 243 |
| 3-5*** | 13.6 | 0 | 0 | 416 | 77.0 | 259 |
| 3-6 | 14.3 | 372* | 0 | 0 | 54.3 | 236 |
| 3-7 | 14.3 | 372 | 0 | 110 | 69.9 | 243 |
| 3-8 | 14.3 | 372 | 0 | 209 | 75.8 | 239 |
| 3-9 | 14.3 | 372 | 0 | 327 | 79.8 | 240 |
| 3-10 | 14.2 | 372 | 0 | 403 | 81.8 | 245 |
| 3-11 | 14.2 | 372 | 0 | 438 | 81.9 | 248 |
| 3-12 | 14.2 | 372 | 0 | 488 | 81.4 | 250 |
| 3-13 | 14.2 | 372 | 0 | 512 | 81.0 | 251 |
| 3-14 | 14.2 | 372 | 0 | 561 | 80.3 | 256 |
| 3-15*** | 13.8 | 186** | 32 | 0 | 61.2 | 232 |

\* 2.0 $\mu$moles/g

\*\* 1.0 $\mu$moles/g

\*\*\* Performance data at 40% oxygen conversion obtained when catalyst had been onstream for 32 $\pm$ 4 hours.

EP 0 266 015 B1

TABLE 3 - CS OPTIMIZATION ON CS ONLY; CS/RE; AND CS/RE+S CATALYSTS (cont'd)

| Experiment No. | %wAg | Re Target Level, ppmw | S Target Level, ppmw | Cs, ppmw (Radiotracer Analysis) | Initial $S_{40}$, % | Initial $T_{40}$, °C |
|---|---|---|---|---|---|---|
| 3-16*** | 13.8 | 186 | 32 | 101 | 70.5 | 235 |
| 3-17*** | 13.8 | 186 | 32 | 208 | 77.2 | 241 |
| 3-18*** | 13.8 | 186 | 32 | 303 | 80.8 | 247 |
| 3-19 | 12.7 | 186 | 32 | 372 | 82.4 | 250 |
| 3-20*** | 13.8 | 186 | 32 | 402 | 83.0 | 253 |
| 3-21 | 12.7 | 186 | 32 | 421 | 82.9 | 253 |
| 3-22 | 12.7 | 186 | 32 | 450 | 82.7 | 255 |
| 3-23*** | 13.8 | 186 | 32 | 515 | 81.7 | 261 |

* 2.0 $\mu$moles/g

** 1.0 $\mu$moles/g

*** Performance data at 40% oxygen conversion obtained when catalyst had been onstream for 32 $\pm$ 4 hours.

EP 0 266 015 B1

TABLE 4 - CS-OPTIMIZED CATALYSTS ON DIFFERENT CARRIERS WITH AND WITHOUT RE/S

| Experiment No. | %wAg | Carrier | Cs, ppmw (Radiotracer Analysis) | Re Target Level ppmw | S Target Level ppmw | Initial $S_{40}$, % | Initial $40$, °C |
|---|---|---|---|---|---|---|---|
| 4-1 | 10.3 | A | 162 | 0 | 0 | 80.1 | 248 |
| 4-2 | 10.6 | A | 234 | 93* | 16* | 82.9 | 262 |
| 4-3 | 14.3 | B | 236 | 0 | 0 | 80.3 | 240 |
| 4-4 | 12.7 | B | 421 | 186** | 32** | 82.9 | 253 |
| 4-5 | 14.1 | C | 256 | 0 | 0 | 80.3 | 240 |
| 4-6 | 14.4 | C | 395 | 186 | 32 | 83.4 | 255 |
| 4-7 | 15.0 | D | 309 | 0 | 0 | 80.9 | 240 |
| 4-8 | 14.9 | D | 482 | 186 | 32 | 84.1 | 260 |
| 4-9 | 14.6 | E | 386 | 0 | 0 | 80.0 | 241 |
| 4-10 | 14.1 | E | 540 | 186 | 32 | 83.3 | 264 |
| 4-11 | 19.0 | F | 637 | 0 | 0 | 80.4 | 235 |
| 4-12 | 18.2 | F | 899*** | 186 | 32 | 81.2 | 241 |

* 0.5 $\mu$moles/g

** 1.0 $\mu$moles/g

*** may not be fully optimized with respect to Cs

TABLE 5 EFFECT OF DIFFERENT RELATIVE AND ABSOLUTE AMOUNTS OF RE AND S ON CESIUM OPTIMIZED CATALYST PERFORMANCE

| Experiment No. | %wAg | Cs, ppmw (Radiotracer Analysis) | Re Target Level, ppmw($\mu$mole/g) | S Target Level, ppmw($\mu$mole/g) | Initial $S_{40}$, % | Initial $T_{40}$, °C |
|---|---|---|---|---|---|---|
| 5-1 | 14.3 | 236 | 0 | 0 | 80.0 | 242 |
| 5-2 | 13.8 | 297 | 93(0.5) | 0 | 80.4 | 246 |
| 5-3 | 13.9 | 360 | 186(1.0) | 0 | 80.6 | 241 |
| 5-4 | 14.2 | 438 | 372(2.0) | 0 | 81.9 | 248 |
| 5-5 | 14.5 | 486 | 465(2.5) | 0 | 82.3 | 248 |
| 5-6 | 14.1 | 567 | 558(3.0) | 0 | 82.5 | 248 |
| 5-7 | 14.0 | 634 | 744(4.0) | 0 | 80.2 | 248 |
| 5-8 | 14.2 | 341 | 0 | 32(1.0) | 80.8 | 243 |
| 5-9 | 12.7 | 421 | 186(1.0) | 32(1.0) | 82.9 | 254 |
| 5-10 | 14.1 | 552 | 372(2.0) | 32(1.0) | 84.3 | 254 |
| 5-11 | 13.8 | 505 | 186(1.0) | 64(2.0) | 82.0 | 273 |
| 5-12 | 12.8 | 513 | 372(2.0) | 64(2.0) | 81.7 | 274 |

EP 0 266 015 B1

## TABLE 6 - OPTIMIZATION WITH DIFFERENT ALKALIS, WITH AND WITHOUT RE AND RE+S

| Experiment No. | %wAg | Alkali Dopant Added | Target ppmw Alkali | Re Target Level, ppmw | S Level ppmw | Target Initial $S_{40}$, % | Initial $T_{40}$, °C |
|---|---|---|---|---|---|---|---|
| 6-1 | 13.6 | None | 0 | 0 | 0 | 74.6 | 229 |
| 6-2 | 14.3 | None | 0 | 372* | 0 | 54.3 | 236 |
| 6-3 | 13.8 | None | 0 | 186** | 32*** | 61.2 | 232 |
| 6-4 | 14.3 | Cs | 230 | 0 | 0 | 80.0 | 242 |
| 6-5 | 14.2 | Cs | 420 | 372 | 0 | 81.9 | 248 |
| 6-6 | 12.7 | Cs | 410 | 186 | 32 | 82.9 | 253 |
| 6-7 | 14.0 | Rb | 170 | 0 | 0 | 79.4 | 238 |
| 6-8 | 14.6 | Rb | 305 | 372 | 0 | 80.0 | 246 |
| 6-9 | 14.3 | Rb | 325 | 186 | 32 | 81.2 | 248 |
| 6-10 | 14.6 | K | 130 | 0 | 0 | 79.4 | 240 |
| 6-11 | 14.5 | K | 200 | 372 | 0 | 78.1 | 239 |

\*   2.0 $\mu$moles/g

\*\* 1.0 $\mu$moles/g

\*\*\* 1.0 $\mu$moles/g

EP 0 266 015 B1

EP 0 266 015 B1

TABLE 6 - OPTIMIZATION WITH DIFFERENT ALKALIS, WITH AND WITHOUT RE AND RE+S (cont'd)

| Experiment No. | %wAg | Alkali Dopant Added | Target ppmw Alkali | Re Target Level, ppmw | S Level ppmw | Target Initial $S_{40}$, % | Initial $T_{40}$, °C |
|---|---|---|---|---|---|---|---|
| 6-12 | 14.1 | K | 250 | 186 | 32 | 78.6 | 241 |
| 6-13 | 14.4 | Na | 207 | 0 | 0 | 76.5 | 234 |
| 6-14 | 14.7 | Na | 92 | 372 | 0 | 74.3 | 246 |
| 6-15 | 14.5 | Na | 253 | 186 | 32 | 75.9 | 241 |
| 6-16 | 13.9 | Li | 40 | 0 | 0 | 74.8 | 233 |
| 6-17 | 14.2 | Li | 120 | 372 | 0 | 63.5 | 239 |
| 6-18 | 13.7 | Li | 100 | 186 | 32 | 68.2 | 234 |
| 6-19 | 13.2 | Cs + Rb | 160 + 110 | 0 | | 79.4 | 245 |
| 6-20 | 13.2 | Cs + Rb | 160 + 195 | 372 | | 81.3 | 256 |

EP 0 266 015 B1

TABLE 7 - EFFECT OF VARIOUS RHENIUM CO-PROMOTERS ON CATALYST PERFORMANCE

| Experiment No. | %wAg | Alkali metal[a) ppmw | Re Target[b) Level, ppmw($\mu$mole/g) | Re Co-promoter (Salt Added) | Co-Promoter Element, Target Level, ppmw($\mu$mole/g) | Initial $S_{40}$, % | Initial $T_{40}$, °C |
|---|---|---|---|---|---|---|---|
| 7-1 | 14.3 | 236 Cs | 0 | None | 0 | 80.0 | 242 |
| 7-2 | 13.9 | 360 Cs | 186(1.0) | None | 0 | 80.6 | 241 |
| 7-3 | 14.2 | 438 Cs | 372(2.0) | None | 0 | 81.9 | 248 |
| 7-4 | 13.3 | 405 Cs[h) | 186(1.0) | $(NH_4)_2SO_4$ | S,32(1.0) | 83.1 | 259 |
| 7-5 | 13.7 | 392 Cs | 186(1.0) | $(NH_4)_2SO_3$ | S,32(1.0) | 82.9 | 250 |
| 7-6 | 12.7 | 421 Cs | 186(1.0) | $(NH_4)_2SO_4$ | S,32(1.0) | 82.9 | 253 |
| 7-7 | 13.8 | 505 Cs | 186(1.0) | $(NH_4)_2SO_4$ | S,64(2.0) | 82.0 | 273 |
| 7-8 | 13.7 | 419 Cs | 186(1.0) | p-toluene-sulfonic acid | S,32(1.0) | 83.6 | 256 |
| 7-9[i) | 13.1 | 394 Cs | 186(1.0) | $(NH_4)_2CrO_4$ | Cr,52(1.0) | 82.9 | 269 |
| 7-10 | 13.9 | 393 Cs | 186(1.0) | $(NH_4)_2Mo_2O_7$ | Mo,96(1.0) | 83.5 | 267 |
| 7-11[i) | 14.1 | 389 Cs | 186(1.0) | $H_2WO_4$ | W,184(1.0) | 83.0 | 259 |
| 7-12 | 13.5 | 338 Cs | 186(1.0) | $KMnO_4$ | Mn,55(1.0) | 80.8 | 242 |
| 7-13 | 13.5 | 391 Cs | 186(1.0) | $NH_4ClO_4$ | Cl,35.5(1.0) | 80.6 | 244 |
| 7-14 | 14.3 | 463 Cs | 186(1.0) | $NH_4VO_3$ | V,51(1.0) | 79.5 | 277 |

EP 0 266 015 B1

TABLE 7 - EFFECT OF VARIOUS RHENIUM CO-PROMOTERS ON CATALYST PERFORMANCE (cont'd)

| Experiment No. | %wAg | Alkali metal[a] ppmw | Re Target[b] Level, ppmw($\mu$mole/g) | Re Co-promoter (Salt Added) | Co-Promoter Element, Target Level, ppmw($\mu$mole/g) | Initial $S_{40}$, % | Initial $T_{40}$, °C |
|---|---|---|---|---|---|---|---|
| 7-15 | 13.8 | 375 Cs | 186(1.0) | $NH_4H_2PO_4$ | P,31(1.0) | 80.4 | 252 |
| 7-16 | 14.5 | 160 K | 186(1.0) | $(NH_4)_6Mo_7O_{24}.4H_2O$ | Mo,96(1.0) | 81.1 | 279 |
| 7-17 | 14.1 | 200 K | 186(1.0) | $H_2WO_4$ | W,184(1.0) | 79.3 | 260 |
| 7-18 | 14.4 | 160 K | 186(1.0) | $(NH_4)_2CrO_4$ | Cr,52(1.0) | 78.9 | 273 |
| 7-19 | 14.6 | 138 Na | 186(1.0) | $(NH_4)_6Mo_7O_{24}.4H_2O$ | Mo,96(1.0) | 75.3 | 257 |
| 7-20 | 14.3 | 255 Rb | 186(1.0) | $(NH_4)_6Mo_7O_{24}.4H_2O$ | Mo,96(1.0) | 82.8[c] | 269[c] |
| 7-21[j] | 14.1 | 138 Cs + 120 K | 186(1.0) | $(NH_4)_6MO_7O_{24}.4H_2O$ | Mo,96(1.0) | 82.6 | 270 |
| 7-22 | 12.9 | 384 Cs + 46 Na[d] | 186(1.0) | $(NH_4)_2SO_4$ | S,64(2.0) | 83.3 | 260 |
| 7-23 | 13.3 | 383 Cs + 46 Na[d] | 186(1.0) | $(NH_4)_2CrO_4$ | Cr,52(1.0) | 83.0 | 269 |
| 7-24 | 13.6 | 404 Cs + 46 Na[d] | 186(1.0) | $H_2WO_4$ | S,32(1.0) W,184(1.0) | 83.8 | 260 |
| 7-25 | 13.4 | 394 Cs + 78 K[d] | 186(1.0) | $(NH_4)_2SO_4$ | S,32(1.0) S,64(2.0) | 83.1 | 258 |
| 7-26 | 14.7 | 387 Cs + 78 K[d] | 186(1.0) | $HWO_4$ | W,184(1.0) | 83.8 | 266 |
| 7-27 | 14.3 | 293 Cs + 7 Li[d] | 186(1.0) | $(NH_4)_2SO_4$ | S,32(1.0) S,32(1.0) | 82.4 | 245 |

TABLE 7 - EFFECT OF VARIOUS RHENIUM CO-PROMOTERS ON CATALYST PERFORMANCE (cont'd)

| Experiment No. | %wAg | Alkali metal[a] ppmw | Re Target[b] Level, ppmw($\mu$mole/g) | Re Co-promoter (Salt Added) | Co-Promoter Element, Target Level, ppmw($\mu$mole/g) | Initial $S_{40}$, % | Initial $T_{40}$, °C |
|---|---|---|---|---|---|---|---|
| 7-28 | 13.7 | 407 Cs + 7 Li[d] + 46 Na[d] | 186(1.0) | $(NH_4)_2SO_4$ | S,64(2.0) | 84.9 | 259 |
| 7-29 | 13.9 | 380 Cs + 28 Li[e] | 186(1.0) | $(NH_4)_2CrO_4$ | Cr,52(1.0) | 83.6 | 272 |
| 7-30 | 13.6 | 380 Cs + 7 Li[e] | 186(1.0) | $(NH_4)_2SO_4$ | S,32(1.0) | 83.6 | 253 |
| 7-31 | 13.3 | 354 Cs + 39 K[f] | 372(2.0)[f] | $(NH_4)_2SO_4$ | S,32(1.0) | 83.1 | 260 |
| 7-32 | 13.7 | 393 Cs + 14 Li[g] | 186(1.0) | $(NH_4)_2SO_4$ | S,32(1.0) | 83.8 | 257 |

a) radiotracer on Cs, target level on other alkali metals, alkali added as hydroxide unless otherwise noted.

b) rhenium added to impregnating solution as $NH_4ReO_4$ unless otherwise noted.

c) 38% conversion data.

d) as sulfate

e) as nitrate

f) 1 $\mu$mole/g each of $NH_4ReO_4$ and $KReO_4$.

g) as $LiBO_2$

h) $CsNO_3$ used rather than CsOH in impregnating solution; target level indicated.

i) may not be fully optimized with respect to cesium.

j) may not be fully optimized with respect to potassium.

Claims

1. A catalyst composition, suitable for the catalytic manufacture of ethylene oxide from ethylene and oxygen, containing silver and a support, characterized in that the catalyst composition comprises a

EP 0 266 015 B1

promoting amount of rhenium or compound thereof, a promoting amount of at least one further metal or compound thereof and that the support has a surface area of less than 20 m²/g.

2. A catalyst as claimed in claim 1, in which the further metal is selected from alkali metals, alkaline earth metals, molybdenum, tungsten, chromium, titanium, hafnium, zirconium, vanadium, thallium, thorium, tantalium, niobium, gallium and germanium.

3. A catalyst as claimed in claim 2, in which the further metal is an alkaline earth metal.

4. A catalyst as claimed in claim 3, in which the further metal is magnesium, barium or calcium.

5. A catalyst as claimed in claim 2, in which the further metal is an alkali metal.

6. A catalyst as claimed in claim 5, in which the further metal is potassium, rubidium or cesium.

7. A catalyst as claimed in claim 6, in which the further metal is cesium.

8. A catalyst as claimed in anyone of claims 1 to 7, in which the support has a surface area of from 0.01 to 10 m²/g.

9. A catalyst as claimed in claim 8, in which the support has a surface area of from 0.05 to 5 m²/g.

10. A catalyst as claimed in claim 9, in which the support has a surface area of from 0.1 to 3 m²/g.

11. A catalyst as claimed in any one of claims 1 to 10, in which the support is alpha-alumina.

12. A catalyst as claimed in any one of claims 1 to 11, which comprises 1 to 30% wt of silver.

13. A catalyst as claimed in claim 12, which comprises 5 to 20% wt of silver.

14. A catalyst as claimed in any one of claims 1 to 13, which comprises 0.01 to 15 mmol rhenium/kg catalyst.

15. A catalyst as claimed in claim 14, which comprises 0.2 to 5 millimol rhenium/kg catalyst.

16. A catalyst as claimed in any one of claims 1 to 15, which comprises 10 to 3000 ppm further metal or metal compound, calculated as metal on weight of catalyst.

17. A catalyst as claimed in claim 16, which comprises 50 to 1000 ppm further metal or metal compound, calculated as metal on weight of catalyst.

18. A catalyst as claimed in any one of claims 1 to 17, which in addition comprises sulphur or a sulphur compound.

19. A catalyst as claimed in claim 18, in which the sulphur content is from 0.2 to 5 millimol/kg catalyst.

20. A catalyst as claimed in any one of claims 1 to 19, which comprises silver, rhenium and cesium.

21. A catalyst as claimed in claim 20, which comprises silver, rhenium, cesium and sulphur.

22. A catalyst as claimed in any one of claims 1 to 20, which comprises silver, rhenium and rubidium.

23. A catalyst as claimed in claim 22, which comprises silver, rhenium, rubidium and sulphur.

24. A catalyst as claimed in claim 22 or 23, which in addition comprises cesium.

25. A catalyst as claimed in any one of claims 1 to 24, and affording a higher selectivity to ethylene oxide at a given oxygen conversion level than is obtained under the same reaction conditions with the same combination of silver, support and none or one of the promotors selected from on the one hand rhenium or compound thereof and on the other hand further metal or compound thereof.

26. A catalyst as claimed in claim 25, and affording a higher selectivity to ethylene oxide at a given oxygen conversion level than is obtained under the same reaction conditions with the same combination of silver, support and a promoter selected from further metal or compound thereof excluding rhenium.

27. A catalyst as claimed in claim 25 or 26, comprising silver, support, rhenium and cesium and affording a higher selectivity to ethylene oxide at a given oxygen conversion level than is obtained under the same reaction conditions with the same combination of silver, support and cesium.

28. A process for the production of ethylene oxide by reacting ethylene with oxygen in vapour phase in the presence of a catalyst composition comprising silver, a support, a promoting amount of rhenium or compound thereof and a promoting amount of at least one further metal or compound thereof.

29. A process for preparing a catalyst composition as claimed in any one of claims 1 to 27 which comprises adding the appropriate amounts of silver, rhenium, further metal and optionally sulphur, to a support.

30. A process as claimed in claim 29, in which the addition is by impregnation.

31. A process as claimed in claim 30, in which the impregnation is coincidental.

32. A process as claimed in claim 30, in which the impregnation is effected in successive steps.

33. A process as claimed in claim 32, in which the first step comprises addition of silver.

34. A process as claimed in claim 33, in which the second step comprises coincidental addition of rhenium and further metal.

35. A process as claimed in claim 33, in which the second step comprises addition of rhenium and further metal is added in a third step.

**Revendications**

1. Une composition catalytique, utilisable pour la production catalytique d'oxyde d'éthylène à partir d'éthylène et d'oxygène, contenant de l'argent et un support, caractérisée en ce que la composition catalytique comprend une quantité activante de rhénium ou d'un composé du rhénium, une quantité activante d'au moins un autre métal ou composé de métal et que le support a une surface spécifique de moins de 20 m$^2$/g.

2. Un catalyseur selon la revendication 1, dans lequel l'autre métal est choisi parmi les métaux alcalins, les métaux alcalino-terreux, le molybdène, le tungstène, le chrome, le titane, le hafnium, le zirconium, le vanadium, le thallium, le thorium, le tantale, le niobium, le gallium et le germanium.

3. Un catalyseur selon la revendication 2, dans lequel l'autre métal est un métal alcalino-terreux.

4. Un catalyseur selon la revendication 3, dans lequel l'autre métal est du magnésium, du baryum ou du calcium.

5. Un catalyseur selon la revendication 2, dans lequel l'autre métal est un métal alcalin.

6. Un catalyseur selon la revendication 5, dans lequel l'autre métal est du potassium, du rubidium ou du césium.

7. Un catalyseur selon la revendication 6, dans lequel l'autre métal est du césium.

8. Un catalyseur selon l'une quelconque des revendications 1 à 7, dans lequel le support a une surface spécifique comprise entre 0,01 et 10 m²/g.

9. Un catalyseur selon la revendication 8, dans lequel le support a une surface spécifique comprise entre 0,05 et 5 m²/g.

10. Un catalyseur selon la revendication 9, dans lequel le support a une surface spécifique comprise entre 0,1 et 3 m²/g.

11. Un catalyseur selon l'une quelconque des revendications 1 à 10, dans lequel le support est de l'alpha-alumine.

12. Un catalyseur selon l'une quelconque des revendications 1 à 11, qui comprend de 1 à 30 % en poids d'argent.

13. Un catalyseur selon la revendication 12, qui comprend de 5 à 20 % en poids d'argent.

14. Un catalyseur selon l'une quelconque des revendications 1 à 13, qui comprend de 0,01 à 15 mmoles de rhénium par kilogramme de catalyseur.

15. Un catalyseur selon la revendication 14, qui comprend de 0,2 à 5 millimoles de rhénium par kilogramme de catalyseur.

16. Un catalyseur selon l'une quelconque des revendications 1 à 15, qui comprend de 10 à 3000 ppm de l'autre métal ou composé de métal.

17. Un catalyseur selon la revendication 16, qui comprend de 50 à 1000 ppm de l'autre métal ou composé de métal, en calculant en métal par rapport au poids du catalyseur.

18. Un catalyseur selon l'une quelconque des revendications 1 à 17, qui comprend en outre du soufre ou un composé du soufre.

19. Un catalyseur selon la revendication 18, dans lequel la teneur en soufre est comprise entre 0,2 et 5 millimoles par kilogramme de catalyseur.

20. Un catalyseur selon l'une quelconque des revendications 1 à 19, qui comprend de l'argent, du rhénium et du césium.

21. Un catalyseur selon la revendication 20, qui comprend de l'argent, du rhénium, du césium et du soufre.

22. Un catalyseur selon l'une quelconque des revendications 1 à 20, qui comprend de l'argent, du rhénium et du rubidium.

23. Un catalyseur selon la revendication 22, qui comprend de l'argent, du rhénium, du rubidium et du soufre.

24. Un catalyseur selon la revendication 22 ou 23, qui comprend en outre du césium.

25. Un catalyseur selon l'une quelconque des revendications 1 à 24, et fournissant une plus haute sélectivité en oxyde d'éthylène à un niveau donné de conversion de l'oxygène que celle obtenue dans les mêmes conditions de réaction avec la même combinaison d'argent, de support et d'aucun ou d'un seul des promoteurs choisis parmi d'un part le rhénium ou un composé du rhénium ou d'autre part l'autre métal ou un composé de ce métal.

26. Un catalyseur selon la revendication 25, et fournissant une plus haute sélectivité en oxyde d'éthylène à un niveau donné de conversion de l'oxygène que celle obtenue dans les mêmes conditions de réaction avec la même combinaison d'argent, de support et d'un promoteur choisi parmi un autre métal ou un composé de ce métal à l'exclusion du rhénium.

27. Un catalyseur selon la revendication 25 ou 26; comprenant de l'argent, un support, du rhénium et du césium et fournissant une plus haute sélectivité en oxyde d'éthylène à un niveau donné de conversion de l'oxygène que celle obtenue dans les mêmes conditions de réaction avec la même combinaison d'argent, de support et de césium.

28. Un procédé pour la production d'oxyde d'éthylène par réaction d'éthylène avec l'oxygène en phase vapeur en présence d'une composition catalytique comprenant de l'argent, un support, une quantité activante de rhénium ou d'un composé du rhénium et une quantité activante d'au moins un autre métal ou composé de métal.

29. Un procédé pour la préparation d'une composition catalytique telle que revendiquée dans l'une quelconque des revendications 1 à 27, qui comprend l'addition des quantités appropriées d'argent, de rhénium, d'un autre métal et éventuellement de soufre à un support.

30. Un procédé selon la revendication 29, dans lequel l'addition est effectuée par imprégnation.

31. Un procédé selon la revendication 30, dans lequel l'imprégnation est simultanée.

32. Un procédé selon la revendication 30, dans lequel l'imprégnation est effectuée en étapes successives.

33. Un procédé selon la revendication 32, dans lequel la première étape comprend l'addition d'argent.

34. Un procédé selon la revendication 33, dans lequel la deuxième étape comprend l'addition simultanée de rhénium et d'un autre métal.

35. Un procédé selon la revendication 33, dans lequel la deuxième étape comprend l'addition de rhénium et l'autre métal est ajouté dans une troisième étape.

**Patentansprüche**

1. Zur katalytischen Herstellung von Ethylenoxid aus Ethylen und Sauerstoff geeignete Katalysatorzusammensetzung mit einem Gehalt an Silber und an einem Träger, dadurch gekennzeichnet, daß die Katalysatorzusammensetzung eine Promotormenge an Rhenium oder Rheniumverbindung, eine Promotormenge an wenigstens einem weiteren Metall oder einer Verbindung hievon umfaßt, und daß der Träger eine Oberfläche von weniger als 20 m²/g aufweist.

2. Katalysator nach Anspruch 1, worin das weitere Metall unter Alkalimetallen, Erdalkalimetallen, Molybdän Wolfram, Chrom, Titan, Hafnium, Zirkon, Vanadium, Thallium, Thorium, Tantal, Niob, Gallium und Germanium ausgewählt ist.

3. Katalysator nach Anspruch 2, worin das weitere Metall ein Erdalkalimetall ist.

4. Katalysator nach Anspruch 3, worin das weitere Metall Magnesium, Barium oder Calcium ist.

5. Katalysator nach Anspruch 2, worin das weitere Metall ein Alkalimetall ist.

6. Katalysator nach Anspruch 5, worin das weitere Metall Kalium, Rubidium oder Cäsium ist.

7. Katalysator nach Anspruch 6, worin das weitere Metall Cäsium ist.

8. Katalysator nach einem der Ansprüche 1 bis 7, worin der Träger eine Oberfläche von 0,01 bis 10 m²/g aufweist.

9. Katalysator nach Anspruch 8, worin der Träger eine Oberfläche von 0,05 bis 5 m²/g aufweist.

10. Katalysator nach Anspruch 9, worin der Träger eine Oberfläche von 0,1 bis 3 m²/g aufweist.

11. Katalysator nach einem der Ansprüche 1 bis 10, worin der Träger alpha-Aluminiumoxid ist.

12. Katalysator nach einem der Ansprüche 1 bis 11, der 1 bis 30 Gew.-% Silber umfaßt.

13. Katalysator nach Anspruch 12, der 5 bis 20 Gew.-% Silber umfaßt.

14. Katalysator nach einem der Ansprüche 1 bis 13, der 0,01 bis 15 mMol Rhenium/kg Katalysator umfaßt.

15. Katalysator nach Anspruch 14, der 0,2 bis 5 Millimol Rhenium/kg Katalysator umfaßt.

16. Katalysator nach einem der Ansprüche 1 bis 15, der 10 bis 3000 ppm an weiterem Metall oder Metallverbindung umfaßt, berechnet als Metall und bezogen auf das Gewicht des Katalysators.

17. Katalysator nach Anspruch 16, der 50 bis 1000 ppm weiteres Metall oder Metallverbindung, berechnet als Metall und bezogen auf das Gewicht des Katalysators, umfaßt.

18. Katalysator nach einem der Ansprüche 1 bis 17, der zusätzlich Schwefel oder eine Schwefelverbindung umfaßt.

19. Katalysator nach Anspruch 18, worin der Schwefelgehalt 0,2 bis 5 Millimol/kg Katalysator beträgt.

20. Katalysator nach einem der Ansprüche 1 bis 19, der Silber, Rhenium und Cäsium umfaßt.

21. Katalysator nach Anspruch 20, der Silber, Rhenium, Cäsium und Schwefel umfaßt.

22. Katalysator nach einem der Ansprüche 1 bis 20, der Silber, Rhenium und Rubidium umfaßt.

23. Katalysator nach Anspruch 22, der Silber, Rhenium, Rubidium und Schwefel umfaßt.

24. Katalysator nach Anspruch 22 oder 23, der zusätzlich Cäsium umfaßt.

25. Katalysator nach einem der Ansprüche 1 bis 24, der eine höhere Selektivität auf Ethylenoxid bei einem gegebenen Sauerstoffumwandlungsgrad erbringt, als sie unter den gleichen Reaktionsbedingungen mit der gleichen Kombination aus Silber, Träger und keinem oder einem der Promotoren, ausgewählt unter einerseits Rhenium oder einer Verbindung hievon und andererseits weiterem Metall oder einer Verbindung hievon, erzielt wird.

26. Katalysator nach Anspruch 25, der eine höhere Selektivität auf Ethylenoxid bei einem gegebenen Sauerstoffumwandlungsgrad erbringt, als sie unter den gleichen Reaktionsbedingungen mit der gleichen Kombination aus Silber, Träger und einem Promotor, ausgewählt unter einem weiteren Metall oder Metallverbindung, jedoch unter Ausschluß von Rhenium, erhalten wird.

27. Katalysator nach Anspruch 25 oder 26, umfassend Silber, Träger, Rhenium und Cäsium, und der eine höhere Selektivität auf Ethylenoxid bei einem gegebenen Sauerstoffumwandlungsgrad erbringt als sie unter den gleichen Reaktionsbedingungen mit der gleichen Kombination aus Silber, Träger und Cäsium erhalten wird.

28. Verfahren zur Herstellung von Ethylenoxid durch Umsetzen von Ethylen mit Sauerstoff in der Gasphase in Gegenwart einer Katalysatorzusammensetzung, die Silber, einen Träger, eine Promotormenge an Rhenium oder einer Verbindung hievon und eine Promotormenge an wenigstens einem weiteren Metall oder einer Verbindung hievon umfaßt.

29. Verfahren zur Herstellung einer Katalysatorzusammensetzung nach einem der Ansprüche 1 bis 27, welches ein Zusetzen der entsprechenden Mengen von Silber, Rhenium, weiterem Metall und gegebenenfalls Schwefel zu einem Träger umfaßt.

30. Verfahren nach Anspruch 29, worin die Zugabe durch Imprägnieren erfolgt.

31. Verfahren nach Anspruch 30, worin die Imprägnierung gleichzeitig erfolgt.

**32.** Verfahren nach Anspruch 30, worin die Imprägnierung in aufeinanderfolgenden Stufen ausgeführt wird.

**33.** Verfahren nach Anspruch 32, worin die erste Stufe die Zugabe von Silber umfaßt.

**34.** Verfahren nach Anspruch 33, worin die zweite Stufe die gleichzeitige Zugabe von Rhenium und weiterem Metall umfaßt.

**35.** Verfahren nach Anspruch 33, worin die zweite Stufe die Zugabe von Rhenium umfaßt und weiteres Metall in einer dritten Stufe zugesetzt wird.

FIG.1

FIG. 2

## FIG.3

## FIG. 4

## FIG.5

## FIG.6

## FIG.7

CARRIER E

PORE VOLUME (cc/g) →
PORE DIAMETER (ANGSTROM) →

## FIG.8

CARRIER F

PORE VOLUME (cc/g) →
PORE DIAMETER (ANGSTROM) →